# EUROPEAN PATENT SPECIFICATION

(11) **EP 4 197 483 B1**
(45) Date of publication and mention of the grant of the patent: **28.01.2026**
(21) Application number: 22196677.3
(22) Date of filing: 20.07.2018
(51) Int. Cl.: A61B 50/31, G16H 40/60, H04W 4/80

(54) **PHYSIOLOGIC MONITORING KITS**
KITS ZUR PHYSIOLOGISCHEN ÜBERWACHUNG
KITS DE SURVEILLANCE PHYSIOLOGIQUE

(30) Priority: 28.07.2017 US 201762538530 P
(43) Date of publication of application: 21.06.2023
(62) Divisional of application: 18838634.6
(73) Proprietor: LifeLens Technologies, Inc., Ivyland, PA 18974 (US)
(72) Inventor: TOTH, Landy, Doylestown, 18901 (US); HARIHARAN, Dhananjai, Doylestown, 18901 (US); SCHWARTZ, Robert, S., Inver Grove Heights, 55076 (US)
(74) Representative: Page White Farrer

(56) References cited:
- US-A1- 2008 097 912
- US-A1- 2014 018 779
- US-A1- 2015 324 538

## Description

### Technical Field

The present disclosure relates to the field of physiologic monitoring and, more particularly, to devices and systems for physiologic monitoring.

### Background

Physiologic monitoring is performed for a range of purposes. Existing technologies, however, are not without shortcomings.

There is a need to measure physiologic parameters of subjects, reliably, simply, and without cables. As the proliferation of mobile and remote medicine increases, simplified and unobtrusive means for monitoring the physiologic parameters of a patient become more important.

Patient compliance is critical to the success of such systems and is often directly correlated to the ease of use and unobtrusiveness of the monitoring solution used.

Existing monitoring systems are often prone to false alarms, usage related failures, unreliable user interfaces, cumbersome interfaces, artifact or electromagnetic interference (EMI) related interference, etc. Such problems decrease productivity of using these systems, can result in lost data, and lead to dissatisfaction on the part of both the subject being monitored and the practitioners monitoring the subject. In the case of a hospital setting, the continual drone of alarms can lead to alarm fatigue and decreased productivity.

Long term compliance of subjects may suffer due to uncomfortable interfaces with monitoring devices, involved maintenance or change-over of disposables, painful or itchy reactions to materials in the devices, and the like.

More reliable, redundant, and user friendly systems are needed that can provide valuable patient data even when operating with limited supervision, expert input, or user manipulation.

US 2014/0018779 discloses a telemedicine care system that connects patients with remote healthcare professionals using a patient care unit at the location of a patient, where the patient care unit provides video, audio, and data communication capabilities by integrating a computing device, various communication devices, and medical instruments into a single unit.

US 2015/0324538 discloses a medical equipment case for containing and transporting at least one article of medical equipment and a two-way audio-visual system, where such equipment is used for telemedicine applications and includes features that provide for security and facilitate return of the medical equipment case and medical equipment and audio-visual system contained therein.

US 2008/0097912 discloses receiving data wirelessly from a medical device, transmitting the data to an intermediary device (such as a properly equipped mobile telephone or personal digital assistant), and formatting a message including the received data for transmission to a medical data server, where the intermediary device includes software configured to receive the data and process the data into a format compatible with the medical data server.

### Summary

There is provided a physiological monitoring kit according to claim 1.

One illustrative, non-limiting objective of this disclosure is to provide systems, devices, methods, and kits for physiologic monitoring of a subject. Another illustrative, non-limiting objective is to provide for physiologic monitoring of a subject over prolonged periods of time, including reliable device functionality for prolonged physiologic monitoring. Another illustrative, non-limiting objective is to provide consistent connections between remote servers and system, devices and kits used in physiologic monitoring of a subject, including during prolonged physiologic monitoring. Yet another illustrative, non-limiting objective is to provide systems, devices, methods and kits for physiologic monitoring of a subject in a remote location where power, and/or local area networks are not readily available.

In some embodiments, the carrying case of the kit comprises a power source coupled to the mounts, the carrying case being configured to recharge a given one of the physiologic monitoring devices when the given physiologic monitoring device is coupled to at least one of the mounts.

In some embodiments, the carrying case of the kit comprises at least a first radio, the carrying case being configured to utilize the first radio for wireless communication with the one or more physiologic monitoring devices to receive monitored physiologic data therefrom. The carrying case of the kit may further comprise a second radio, the carrying case being configured to utilize the second radio for wireless communication with at least one of a satellite and a radio tower to send the monitored physiologic data from the one or more physiologic monitoring devices to a remote server. The carrying case may further comprise a subscriber identity module for a cellular network, the carrying case being configured to utilize the subscriber identity module for wireless communication over the cellular network to send the monitored physiologic data from the one or more physiologic monitoring devices to a remote server.

In some embodiments, the mounts of the carrying case are disposed within one or more of the compartments of the carrying case, the one or more compartments configured to be sealed such that the mounts are isolated from a surrounding environment.

In some embodiments, one or more of the compartments of the carrying case comprises a lid, at least a portion of an interior face of the lid being lined with a mirror such that a subject holding the carrying case can view an attachment site on a body of the subject when attaching one or more of the subject interfaces to the attachment site or when attaching one or more of the physiologic monitoring devices to one or more of the subject interfaces.

In some embodiments, the carrying case of the kit comprises two or more electrodes disposed on an outer surface thereof, the two or more electrodes being electrically coupled to the one or more mounts such that one or more of the physiologic monitoring devices are coupled to at least one of the two or more electrodes when the one or more physiologic monitoring devices are attached to the one or more mounts.

In some embodiments, the carrying case of the kit comprises a microphone and a speaker, and the carrying case is configured to utilize the microphone and the speaker to receive communications and provide feedback to a subject during use.

In some embodiments, the kit further comprises a therapeutic device, at least one of the carrying case and one or more of the physiologic monitoring devices comprising a radio configured for communication with the therapeutic device.

### Brief Description of the Drawings

Several aspects of the disclosure can be better understood with reference to the following drawings. In the drawings, like reference numerals designate corresponding parts throughout the several views.
FIG. 1 illustrates aspects of a modular physiologic monitoring system, according to an embodiment of the invention.
FIGS. 2a and 2b illustrate a carrying case in closed and open positions, respectively, according to an embodiment of the invention.
FIGS. 3a and 3b illustrate another carrying case in closed and open positions, respectively, according to an embodiment of the invention.
FIGS. 4a and 4b illustrate another carrying case in closed and open positions, respectively, according to an embodiment of the invention.
FIG. 5 illustrates a carrying case with a mirror on an interior of a lid thereof, according to an embodiment of the invention.
FIG. 6 illustrates a carrying case having external electrodes, according to an embodiment of the invention.
FIG. 7 illustrates a block diagram of components of a carrying case, according to an embodiment of the invention.
FIG. 8 illustrates a block diagram of components of another carrying case, according to an embodiment of the invention.
FIG. 9 illustrates a system including a case-based gateway, according to an embodiment of the invention.
FIGS. 10a-10m illustrate handoffs between a case-based gateway and a phone-based gateway, according to an embodiment of the invention.
FIGS. 11a-11d illustrate aspects of a carrying case, according to an embodiment of the invention.
FIGS 12a-12d illustrate aspects of another carrying case, according to an embodiment of the invention.
FIG. 13 illustrates a flow diagram of a process for utilizing a carrying case as a gateway, according to an embodiment of the invention.

### Detailed Description

Particular embodiments of the present disclosure are described herein below with reference to the accompanying drawings; however, the disclosed embodiments are merely examples of the disclosure and may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals may refer to similar or identical elements throughout the description of the figures.

There is a need to monitor a subject over prolonged periods of time, such as over several days, weeks, months, etc. To facilitate such prolonged monitoring, there is a need to provide reliable device functionality over such prolonged periods of time. Data or information, such as physiologic parameters of the subject or information derived therefrom, may be continuously or periodically transferred or uploaded to a remote server during a prolonged monitoring period. Thus, there is a need to provide a consistent and reliable connection or means to a remote server over a monitoring period. In some instances, a subject may be in a remote location during prolonged monitoring where power, a local area network, or other resources needed for such connection to a remote server are not readily available. Thus, there is a need to provide means for storing monitoring data while a subject is in such a remote location. Moreover, there is a need to provide a simple, reliable and easy to use monitoring solution for prolonged monitoring of a subject.

A modular physiologic monitoring system in accordance with the present disclosure for assessing one or more physiologic parameters of a subject (e.g., a human subject, a patient, an athlete, a trainer, an animal, such as equine, canine, porcine, bovine, etc.) with a body may include one or more patches, each patch adapted for attachment to the body of the subject (e.g., attachable to the skin thereof, reversibly attachable, adhesively attachable, with a disposable interface and a reusable module, etc.). In aspects, the physiologic monitoring system may include one or more modules, and each module may include a power source (e.g., a battery, a rechargeable battery, an energy harvesting transducer, microcircuit, and an energy reservoir, a thermal gradient harvesting transducer, a kinetic energy harvesting transducer, a radio frequency energy harvesting transducer, a fuel cell, a biofuel cell, etc.), signal conditioning circuitry, processor, communication circuitry, one or more sensors, or the like, configured to generate one or more signals (e.g., physiologic and/or physical signals), stimulus, etc. Such modules may also be referred to herein as hubs.

One or more of the patches may include one or more interconnects, configured and dimensioned so as to couple with one or more of the modules, said modules including a complimentary interconnect configured and dimensioned to couple with the corresponding patch. The patch may include a bioadhesive interface for attachment to the subject, the module retainable against the subject via interconnection with the patch.

In aspects, the patch may be configured so as to be single use (e.g., disposable). The patch may include a thin, breathable, stretchable laminate. In aspects, the laminate may include a substrate, a bioadhesive, one or more sensing or stimulating elements in accordance with the present disclosure, and one or more interconnects for coupling one or more of the sensing elements with a corresponding module.

In aspects, to retain a high degree of comfort and long term wear-ability of the patch on a subject, to limit interference with normal body function, to limit interference with joint movement, or the like, the patch may be sufficiently thin and frail, such that it may not substantially retain a predetermined shape while free standing. Such a definition is described in further detail below. The patch may be provided with a temporary stiffening film to retain the shape thereof prior to placement of the patch onto the body of a subject. Once adhered to the subject, the temporary stiffening film may be removed from the patch. While the patch is adhered to the subject, the shape and functionality of the patch may be substantially retained. Upon removal of the patch from the subject, the now freestanding patch is sufficiently frail such that the patch can no longer substantially retain the predetermined shape (e.g., sufficiently frail such that the patch will not survive in a free standing state). In aspects, stretch applied to the patch while removing the patch from the subject may result in snap back once the patch is in a freestanding state that renders such a patch to crumple into a ball and no longer function.

In aspects, the patch may include a film (e.g., a substrate), with sufficiently high tear strength, such that, as the patch is peeled from the skin of a subject, the patch does not tear. In aspects, the ratio between the tear strength of the patch and the peel adhesion strength of the patch to skin (e.g., tear strength: peel adhesion strength), is greater than 8:1, greater than 4:1, greater than 2: 1, or the like. Such a configuration may be advantageous so as to ensure the patch may be easily and reliably removed from the subject after use without tearing.

In aspects, the patch may include a bioadhesive with peel tack to mammalian skin of greater than 0.02 Newtons per millimeter (N/mm), greater than 0.1N/mm, greater than 0.25N/mm, greater than 0.50N/mm, greater than 0.75N/mm, greater than 2N/mm, or the like. Such peel tack may be approximately determined using an American Society for Testing and Materials (ASTM) standard test, ASTM D3330: Standard test method for peel adhesion of pressure-sensitive tape.

In aspects, the patch may exhibit a tear strength of greater than 0.5N/mm, greater than 1N/mm, greater than 2N/mm, greater than 8N/mm, or the like. Such tear strength may be approximately determined using an ASTM standard test, ASTM D624: Standard test method for tear strength of conventional vulcanized rubber and thermoplastic elastomers.

In aspects, the patch may be provided with a characteristic thickness, of less than 50 micrometers (µm), less than 25µm, less than 12µm, less than 8µm, less than 4µm, or the like. Yet, in aspects, a balance between the thickness, stiffness, and tear strength may be obtained so as to maintain sufficiently high comfort levels for a subject, minimizing skin stresses during use (e.g., minimizing skin stretch related discomfort and extraneous signals as the body moves locally around the patch during use), minimizing impact on skin health, minimizing risk of rucking during use, and minimizing risk of maceration to the skin of a subject, while limiting risk of tearing of the patch during removal from a subject, etc.

In aspects, the properties of the patch may be further altered so as to balance the hydration levels of one or more hydrophilic or amphiphilic components of the patch while attached to a subject. Such adjustment may be advantageous to prevent over hydration or drying of an ionically conducting component of the patch, to manage heat transfer coefficients within one or more elements of the patch, to manage salt retention into a reservoir in accordance with the present disclosure, and/or migration during exercise, to prevent pooling of exudates, sweat, or the like into a fluid measuring sensor incorporated into the patch or associated module, etc. In aspects, the patch or a rate determining component thereof may be configured with a moisture vapor transmission rate of between 200 grams per meter squared per 24 hours (g/m²/24hrs) and 20,000g/m²/24hrs, between 500g/m²/24hrs and 12,000g/m²/24hrs, between 2,000g/m²/24hrs and 8,000g/m²/24hrs, or the like.

Such a configuration may be advantageous for providing a comfortable wearable physiologic monitor for a subject, while reducing material waste and/or cost of goods, preventing contamination or disease spread through uncontrolled re-use, and the like.

In aspects, one or more patches and/or modules may be configured for electrically conducting interconnection, inductively coupled interconnection, capacitively coupled interconnection, with each other. In the case of an electrically conducting interconnect, each patch and module interconnect may include complimentary electrically conducting connectors, configured and dimensioned so as to mate together upon attachment. In the case of an inductively or capacitively coupled interconnect, the patch and module may include complimentary coils or electrodes configured and dimensioned so as to mate together upon attachment.

Each patch or patch-module pair may be configured as a sensing device to monitor one or more local physiologic and/or physical parameters of the attached subject (e.g., local to the site of attachment, etc.), local environment, combinations thereof, or the like, and to relay such information in the form of signals to a host device (e.g., via a wireless connection, via a body area network connection, or the like), one or more patches or modules on the subject, or the like. Each patch and/or patch-module pair may also or alternatively be configured as a stimulating device to apply a stimulus to the subject in response to signaling from the host device, the signaling being based on analysis of the physiologic and/or physical parameters of the subject measured by the sensing device(s).

In aspects, the host device may be configured to coordinate information exchange to/from each module and/or patch, and to generate one or more physiologic signals, physical signals, environmental signals, kinetic signals, diagnostic signals, alerts, reports, recommendation signals, commands, combinations thereof, or the like for the subject, a user, a network, an electronic health record (EHR), a database (e.g., as part of a data management center, an EHR, a social network, etc.), a processor, combinations thereof, or the like.

In aspects, a system in accordance with the present disclosure may include a plurality of substantially similar modules (e.g., generally interchangeable modules, but with unique identifiers), for coupling with a plurality of patches, each patch, optionally different from the other patches in the system (e.g., potentially including alternative sensors, sensor types, sensor configurations, electrodes, electrode configurations, etc.). Each patch may include an interconnect suitable for attachment to an associated module. Upon attachment of a module to a corresponding patch, the module may validate the type and operation of the patch to which it has been mated. In aspects, the module may then initiate monitoring operations on the subject via the attached patch, communicate with one or more patches on the subject, a hub, etc. The data collection from each module may be coordinated through one or more modules and/or with a host device in accordance with the present disclosure. The modules may report a time stamp along with the data in order to synchronize data collection across multiple patch-module pairs on the subject, between subjects, etc. Thus, if a module is to be replaced, a hot swappable replacement (e.g., replacement during a monitoring procedure) can be carried out easily by the subject, a caregiver, practitioner, etc. during the monitoring process. Such a configuration may be advantageous for performing redundant, continuous monitoring of a subject, and/or to obtain spatially relevant information from a plurality of locations on the subject during use.

In aspects, the modules and/or patches may include corresponding interconnects for coupling with each other during use. The interconnects may include one or more connectors, configured such that the modules and patches may only couple in a single unique orientation with respect to each other. In aspects, the modules may be color coded by function. A temporary stiffening element attached to a patch may include instructions, corresponding color coding, etc. so as to assist a user or subject with simplifying the process of monitoring.

In addition to physiologic monitoring, one or more patches and/or modules may be used to provide a stimulus to the subject.

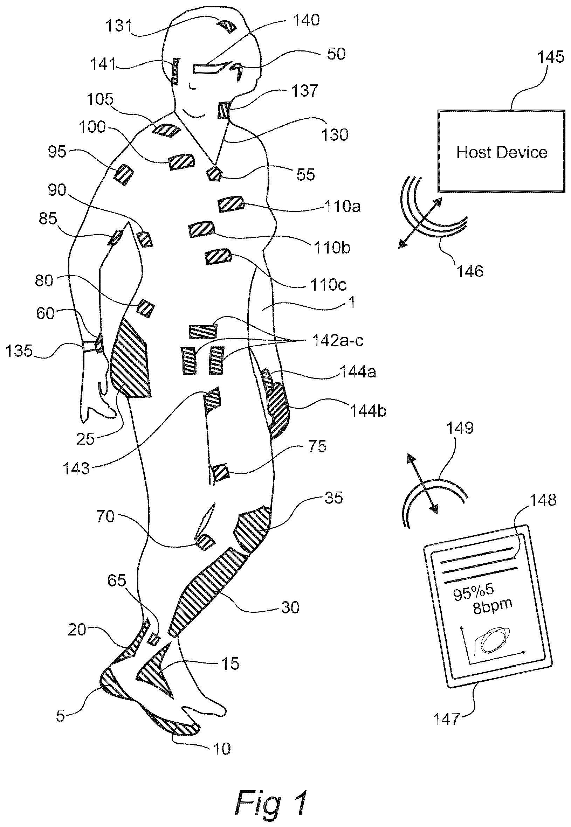

A modular physiologic monitoring system, in some embodiments, includes one or more sensing devices, which may be placed or attached to one or more sites on the subject. Alternatively or additionally, one or more sensing devices may be placed "off" the subject, such as one or more sensors (e.g., cameras, acoustic sensors, etc.) that are not physically attached to the subject. The sensing devices are utilized to establish whether or not an event is occurring and to determine one or more characteristics of the event by monitoring and measuring physiologic parameters of the subject. The determination of whether an event has occurred or is occurring may be made by a device that is at least partially external and physically distinct from the one or more sensing devices, such as a host device in wired or wireless communication with the sensing devices as described below with respect to FIG. 1. The modular physiologic monitoring system includes one or more stimulating devices, which again may be any combination of devices that are attached to the subject or placed "off" the subject, to apply a stimulus to the subject to treat the event, to prevent the event from transition from a first form into a second form, to interrupt the event, to stimulate a type of input to the subject with an alternative form of energy (e.g., stimulating one or more of thermal input, vibration input, mechanical input, a compression or the like with an electrical input), etc.

The sensing devices of a modular physiologic monitoring system, such as patch-module pairs described below with respect to FIG. 1, may be used: to monitor one or more physiologic functions or parameters of a subject; to monitor one or more disease states of a subject; to monitor the state of one or more tissue sites (e.g., tissue health, pressure applied to the tissue, etc.) of a subject; to monitor one or more orientations of a region of a subject with respect to gravity, with respect to one or more other regions of the body of the subject (e.g., back posture, back orientation, neck orientation, spinal rotation, hip rotation, neck rotation, etc.); to monitor any of the above in combination with postural information (e.g., monitoring local muscle activity or spasm in combination with postural information), etc. The sensing devices of the modular physiologic monitoring system, or a host device configured to receive data or measurements from the sensing devices, may be utilized to monitor for one or more events (e.g., through analysis of signals measured by the sensing devices, from metrics derived from the signals, etc.). The stimulating devices of the modular physiologic monitoring system may be configured to deliver one or more stimuli (e.g., electrical, vibrational, acoustic, visual, etc.) to the subject. The stimulating devices may receive a signal from one or more of the sensing devices or a host device, and provide the stimulation in response to the received signal.

FIG. 1 shows aspects of a modular physiologic monitoring system in accordance with the present disclosure. FIG. 1 shows a subject 1 with a series of patches and/or patch-module pairs each in accordance with the present disclosure attached to the subject 1 at sites described below, a host device 145 in accordance with the present disclosure, a feedback/user device 147 in accordance with the present disclosure displaying some data 148 based upon signals obtained from the subject 1, and one or more feedback devices 135, 140, in accordance with the present disclosure configured to convey to the subject 1 one or more aspects of the signals or information gleaned therefrom. In some embodiments, the feedback devices 135, 140 may also or alternatively function as stimulating devices. The host device 145, the user device 147, the patches and/or patch-module pairs, and/or the feedback devices 135, 140 may be configured for wireless communication 146, 149 during a monitoring session.

In aspects, a patch-module pair may be adapted for placement almost anywhere on the body of a subject 1. As shown in FIG. 1, some sites may include attachment to the cranium or forehead 131, the temple, the ear or behind the ear 50, the neck, the front, side, or back of the neck 137, a shoulder 105, an upper arm 95, a chest region with minimal muscle mass 100, integrated into a piece of ornamental jewelry 55 (may be a host, a hub, a feedback device, etc.) on a necklace 130, arrangement on the torso 110a-c, arrangement on the abdomen 80 for monitoring movement or breathing, below the rib cage 90 for monitoring respiration (generally on the right side of the body to substantially reduce EKG influences on the measurements), on a muscle such as a bicep 85, on a wrist 135 or in combination with a wearable computing device 60 on the wrist (e.g., a smart watch, a fitness band, etc.), on a buttocks 25, on a thigh 75, on a calf muscle 70, on a knee 35 particularly for proprioception based studies and impact studies, on a shin 30 primarily for impact studies, on an ankle 65, over an Achilles tendon 20, on the front or top of the foot 15, on a heel 5, or around the bottom of a foot or toes 10. Other sites for placement of such devices are envisioned. Selection of the monitoring and/or stimulating sites is generally determined based upon the intended application of the patch-module pairs described herein.

Additional placement sites on the abdomen, perineal region 142a-c, genitals, urogenital triangle, anal triangle, sacral region, inner thigh 143, or the like may be advantageous in the assessment of autonomic neural function of a subject. Such placements regions may be advantageous for assessment of peripheral nervous system (PNS) activity, somatosensory function, assessment of sympathetic nervous system (SNS) functionality, etc.

Placement sites on the wrist 144a, hand 144b or the like may advantageous for interacting with a subject, such as via performing a stress test, performing a thermal stress test, performing a tactile stress test, monitoring outflow, afferent traffic, efferent traffic, etc.

Placement sites on the nipples, areola, lips, labia, clitoris, penis, the anal sphincter, levator ani muscle, over the ischiocavernous muscle, deep transverse perineal muscle, labium minus, labium majus, one or more nerves near the surface thereof, posterior scrotal nerves, perineal membrane, perineal nerves, superficial transverse perineal nerves, dorsal nerves, inferior rectal nerves, etc. may be advantageous for assessment of autonomic neural ablation procedures, autonomic neural modulation procedures, assessment of the PNS of a subject, assessment of sexual dysfunction of a subject, etc.

Placement sites on the face 141, over ocular muscles, near the eye, over a facial muscle (e.g., a nasalis, temporalis, zygonaticus minor/major, orbicularis oculi, occipitofrontalis), near a nasal canal, over a facial bone (e.g., frontal process, zygomatic bone/surface, zygomaticofacial foreman, malar bone, nasal bone, frontal bone, maxilla, temporal bone, occipital bone, etc.), may be advantageous to assess ocular function, salivary function, sinus function, interaction with the lips, interaction with one or more nerves of the PNS (e.g., interacting with the vagus nerve within, on, and/or near the ear of the subject), etc.

In aspects, a system in accordance with the present disclosure may be configured to monitor one or more physiologic parameters of the subject 1 before, during, and/or after one or more of, a stress test, consumption of a medication, exercise, a rehabilitation session, a massage, driving, a movie, an amusement park ride, sleep, intercourse, a surgical, interventional, or non-invasive procedure, a neural remodeling procedure, a denervation procedure, a sympathectomy, a neural ablation, a peripheral nerve ablation, a radio-surgical procedure, an interventional procedure, a cardiac repair, administration of an analgesic, a combination thereof, or the like. In aspects, a system in accordance with the present disclosure may be configured to monitor one or more aspects of an autonomic neural response to a procedure, confirm completion of the procedure, select candidates for a procedure, follow-up on a subject after having received procedure, assess the durability of a procedure, or the like (e.g., such as wherein the procedure is a renal denervation procedure, a carotid body denervation procedure, a hepatic artery denervation procedure, a lower urinary tract symptoms (LUTs) treatment, a bladder denervation procedure, a urethral treatment, a prostate ablation, a prostate nerve denervation procedure, a cancer treatment, a pain block, a neural block, a bronchial denervation procedure, a carotid sinus neuromodulation procedure, implantation of a neuromodulation device, tuning of a neuromodulation device, etc.).

Additional details regarding modular physiologic monitoring systems, kits and methods are further described in PCT application serial no. PCT/US2014/041339, published as WO 2014/197822 and titled "Modular Physiologic Monitoring Systems, Kits, and Methods," PCT application serial no. PCT/US2015/043123, published as WO 2016/019250 and titled "Modular Physiologic Monitoring Systems, Kits, and Methods," and PCT application serial no. PCT/US2017/030186, titled "Monitoring and Management of Physiologic Parameters of a Subject," the disclosures of which are incorporated by reference herein in their entirety.

Modular physiologic monitoring systems, as discussed above, may include or utilize a number of sensing and/or stimulating devices, such as in the form of patch-module pairs. Described herein is a full self-contained kit for physiologic monitoring, providing a system configured to monitor one or more properties of a subject and to transfer data associated with or derived from such properties to a remote location for analysis. In some embodiments, such a kit or system includes a carrying case, one or more hubs or modules, and one or more patches.

Advantageously, carrying cases in some embodiments allow a user to have a complete, self-contained product that is suitable for single patient use (e.g., very simply on-boarding of a patient as the carrying case mitigates or avoids issues relating to a user's smartphone or other device meeting minimal specification, the user not being capable of setting up the smartphone or other device to route data out to a cloud-based service or other remote server, etc.). Carrying cases in some embodiments further provide a kit for physiologic monitoring as a complete stand-alone product for deployment in remote areas, such that the kit may be deployed into the field and the user has everything they need to get monitored physiologic data out to a cloud-based service or other remote server without any additional hardware or power source.

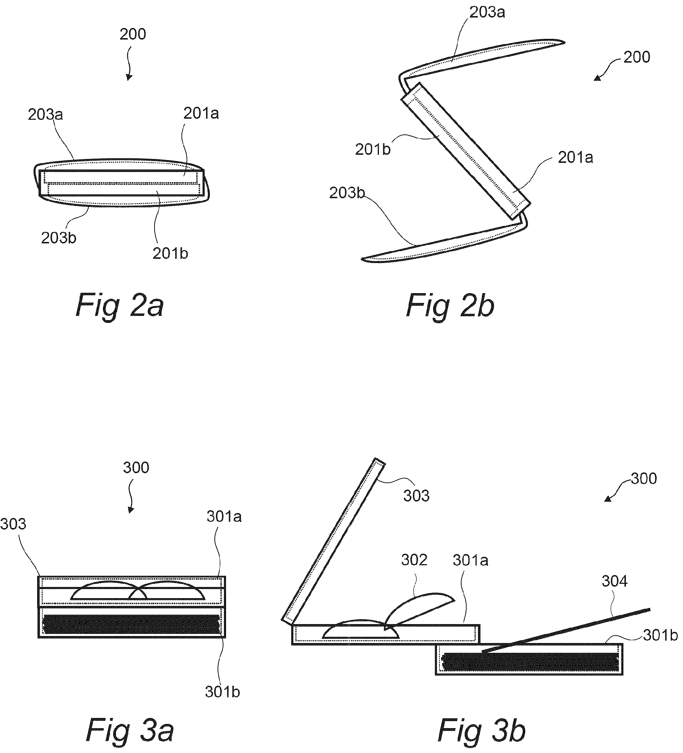

FIG. 2a illustrates an example of a carrying case 200 in a closed position. The carrying case 200 includes chambers 201a, 201b and lids 203a, 203b. In some embodiments, chamber 201a provides a repository for one or more hubs or modules while chamber 201b provides a repository for one or more patches. The lids 203a, 203b facilitate access to the chambers 201a, 201b, as illustrated by the view of the carrying case 200 in an open position in FIG. 2b. While FIG. 2b shows both of the lids 203a, 203b in an opened position, each lid 203a, 203b may be opened or closed independently in some embodiments, such that one or both of the lids 203a, 203b may be opened simultaneously. In other embodiments, the lids 203a, 203b may be configured such that only one may be opened at a given time.

It is important to note that while FIGS. 2a and 2b and other figures described herein illustrate carrying cases with two chambers, embodiments are not limited to this arrangement and a carrying case may have more than two chambers or fewer than two chambers. A carrying case with a single chamber may be desired for a physiologic monitoring kit or system in which monitoring or stimulating devices are a single piece rather than multi-part (e.g., such as the disposable patch and reusable module arrangement of certain patch-module pairs described herein). A carrying case with more than two chambers may be desired for a physiologic monitoring kit or system which uses multiple different types of patches and/or modules (e.g., different types of modules configured for attachment to a single type of patch, a single type of module configured for attachment to different types of patches, combinations thereof, etc.) or more generally multiple types of devices (e.g., different types of devices configured for attachment to different sites on a subject, configured for monitoring different types of physiologic parameters, configured for use in different types of environments such as hot/cold, wet/dry, etc.).

FIGS. 3a and 3b show an alternate arrangement of a carrying case 300, which again includes multiple chambers 301a, 301b but only a single lid 303. Whereas the lids 203a, 203b of carrying case 200 are clamshell-like and open to provide access to the respective chambers 201a, 201b, the lid 303 of carrying case 300 opens to reveal chamber 301a, while the chambers 301a, 301b are slidingly coupled to provide access to the chamber 301b. FIGS. 3a and 3b show a set of modules 302 stored in the chamber 301a, and a set of patches 304 stored in the chamber 301b.

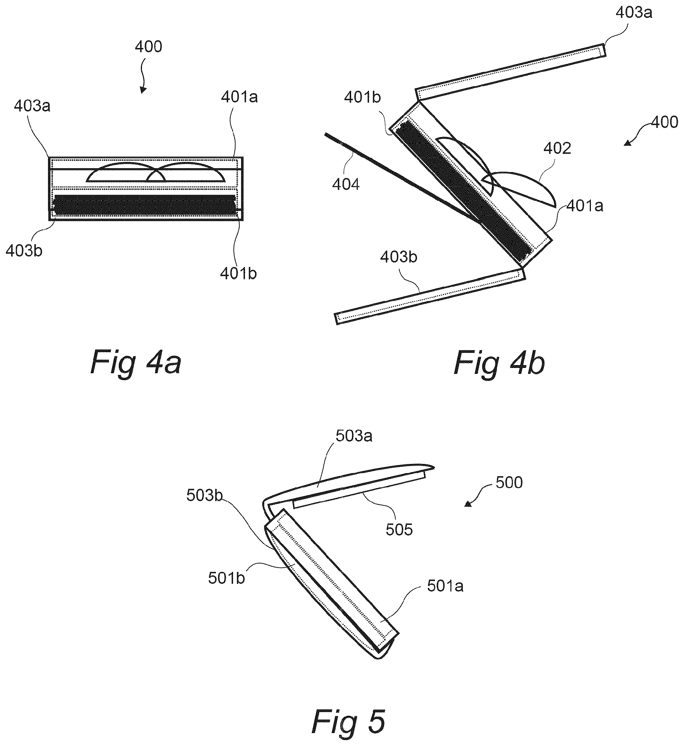

FIGS. 4a and 4b show another arrangement of a carrying case 400, again including multiple chambers 401a, 401b and multiple lids 403a, 403b. The lids 403a, 403b are connected via hinges and open to provide access to the chambers 401a, 401b. As shown, the chamber 401a stores a number of modules 402 and the chamber 401b stores a number of patches 404.

It is to be appreciated that numerous other types and arrangements of chambers and lids or other access means for chambers of a carrying case may be used in other embodiments. A carrying case may be segmented or designed in various other ways to store patches, hubs or more generally devices or parts thereof for physiologic monitoring. As will be described in further detail below, various hardware may be integrated into carrying cases such as carrying cases 200, 300 and 400 for charging hubs or other devices or parts thereof, for providing a gateway to a remote server, etc. In some embodiments, a carrying case provides recharge functionality to one or more hubs when the hubs are mounted in the carrying case. Providing recharge functionality may facilitate hot-swapping, such as hot-swapping using techniques described in U.S. Provisional Patent Application Serial No. 62/425,994, filed November 23, 2016 and titled "Continuous Long-Term Monitoring of a Subject," the disclosure of which is incorporated by reference herein in its entirety.

The carrying case may also or alternatively function as a gateway. For example, the carrying case may be part of a system or kit that includes one or more hubs configured to wirelessly communicate data on a body area network (BAN) and to a gateway. The gateway may facilitate communication between hubs in the BAN, to and from one or more remote servers, etc. In some embodiments, the carrying case functions as a gateway, allowing one or more hubs or other devices in the BAN to upload, transfer or otherwise communicate physiologic data to the remote server, to devices in the BAN, etc. In some cases, the gateway may be configured to relay commands or instructions from the remote server to one or more devices in the BAN, such as instructions to apply a stimulus in response to a detected event.

Carrying cases may be made small, such that a subject or user could keep the carrying case on their person (e.g., in a pocket, wallet, purse, briefcase or other bag, etc.) or by their bedside in an unobtrusive manner. This illustratively allows for devices used in physiologic monitoring to hand off between using the carrying case as a gateway and using alternate gateways (e.g., such as a user's smartphone, tablet or other mobile device). In some embodiments, carrying cases can be oriented to sit on a nightstand or other furniture for bedside use. In such an arrangement, the carrying case may be near the subject during sleep and acts as the gateway during such times. The carrying case may hand off the gateway role to another device (e.g., the user's smartphone, a personal assistant-based device, a smart appliance, an IoT device, or the like) during remote use not near the bed of the subject if desired. Handoffs between the carrying case and other gateways, such as the user's smartphone or a personal assistant-based device, may be made during use so as to seamlessly continue monitoring the subject as the subject moves around in an environment.

In some embodiments, different devices are assigned different priorities as gateways. In one non-limiting example, the carrying case is designated as a backup gateway relative to another device such as the user's smartphone designated as a primary gateway. Therefore, when both the carrying case and the user's smartphone are in range of the BAN, the user's smartphone would be selected as the gateway. If the user's smartphone is out of range, devices in the BAN would switch to using the carrying case as the gateway. Alternatively, the carrying case may be designated as the primary gateway. Of course, various other arrangements are possible, including hierarchies of potential gateways including a primary gateway and multiple backup gateways of varying priority. The multiple backup gateways may, in some embodiments, include one or more devices present in a home or other operating environment of the user.

In some embodiments, the choice of gateway may be based on one or more factors other than proximity to devices in the BAN. For example, one such factor is the charge level or battery level of different potential gateways. If a user's smartphone is fully charged, it may be used as the preferred gateway relative to the carrying case. If the user's smartphone reaches a threshold charge level, such as 50%, then the carrying case may be the preferred gateway. It is to be appreciated that this threshold and other thresholds described herein are provided by way of example only, and that the particular values of such thresholds may be set as a matter of design choice for a particular implementation unless otherwise specified.

Another factor which may be used for the choice of gateway is a wireless signal strength of the connection between devices in the BAN and the potential gateway, where potential gateways would be selected based on which has a higher wireless signal strength. Yet another factor which may be used for the choice of gateway is an available storage capacity of the potential gateways, where potential gateways would be selected based on which has a higher available storage capacity (e.g., such that the gateway may function as a backup for monitored physiologic data, or may cache monitored physiologic data in the event that a connection to a remote server is unavailable). Other factors include a connection status to location (e.g., depending on whether the user is at home, work, a doctor's office, etc. different gateways may be preferred), the status of a remote server (e.g., if a remote server is down or otherwise unavailable, a gateway such as a carrying case with designated storage available for caching or backing up monitored physiologic data may be preferred to a pass-through gateway that does not have storage available for caching or backing up), security level (e.g., a carrying case may be designed as proprietary with specialized secure network protocols, which may be preferred relative to a device such as a user's smartphone which uses open communication protocols that may be more open to attack), etc. As will be described in further detail below, the carrying case may be programmed or otherwise configured to make selection decisions for handing off to and from other potential gateways based on combinations of one or more of the above-described and other factors.

In some embodiments, the carrying case may include a battery or other power source with sufficient energy storage capacity to monitor a subject for the expected duration of a monitoring session, such as greater than one week, greater than two weeks, greater than one month, or the like. The carrying case may provide the capacity to charge one or more hubs more than 5 times, more than 10 times, more than 20 times or the like before the carrying case needs to be recharged. The energy storage capacity of a carrying case, as well as the data storage capacity (e.g., for caching monitored physiologic data in the event that connection to a remote server is unavailable), may vary based on expected usage scenario. In some usage scenarios, such as in military, remote and cardiac monitoring applications, the expected monitoring session may have a duration ranging from a week to greater than one month.

In some embodiments, the carrying case may include an energy harvesting system, such as a kinetic energy harvesting system, a thermal gradient energy harvesting system, a solar energy harvesting system, etc. In such embodiments, the power source included in the carrying case may be recharged over time using the energy harvesting system for extended use. Such embodiments may thus permit use of the carrying case in certain climates or other operating environments where direct power access is limited.

Carrying cases in some embodiments are configured to function as secondary packaging on a store shelf, a hospital or office setting, etc. Carrying cases may be designed to be stackable or inter-lockable, such that a collection of carrying cases may be efficiently stored on a shelf in a store prior to purchase, in a hospital or doctor's office, etc.

The carrying cases may be part of kits that include the carrying case and one or more devices used in physiologic monitoring, such as one or more hubs and one or more patches, a watch or other wearable device, etc. In some embodiments, the carrying case may include a watch recharge mount, which may be useful for fitness applications wherein a user would like to keep everything together in a bag, but take only the bare minimum hardware during a workout (e.g., the watch, one or more hubs, one or more patches, etc.). The carrying case advantageously keeps all components charged and ready to go. The needed components are simply attached to the subject during use and stored in the carrying case otherwise.

In some embodiments, a carrying case may provide a self-contained gateway for a kit, including by way of example a dedicated subscriber identity module (SIM) for one or more designated wireless carriers. Alternatively or additionally, the carrying case may be provided with a network interface card or other hardware configured for communication on wireless local area network (WLAN) networks, Internet of Things (IoT) networks, short range wireless networks such as radio frequency identification (RFID), near-field communication (NFC), Bluetooth^{®}, etc.

In some embodiments, a kit may include everything that is needed to perform a particular type of monitoring on a subject. Some non-limiting examples include kits for monitoring an electrocardiogram (ECG) of a subject, kits for monitoring a 12-lead ECG of a subject, kits for monitoring a disease state of a subject, kits for monitoring gastrointestinal (GI) motility of a subject, kits for monitoring a sleep state of a subject (e.g., of an infant), kits for fetal monitoring, kits for monitoring a subject during labor, kits for monitoring sleep habits of a subject, kits for monitoring an electromyogram (EMG) of a subject (e.g., including kits for monitoring specific EMGs dependent on target muscle groups, indications, sports, etc.), kits for monitoring breathing of a subject, kits for monitoring a gait of a subject, kits for monitoring a state of anesthesia of a subject (e.g., before, during or after a surgical or other procedure), kits for monitoring a blood oxygen level of a subject, kits for monitoring resting ECG of a subject, kits for monitoring cardiac parameters of a subject, kits for monitoring fitness of a subject, kits customized for monitoring particular sports or other activities, kits for monitoring one or more combinations of the above, or the like.

In some embodiments, kits for different purposes may differ in various ways. In some embodiments, the carrying case doubles as a secondary packaging for the kit, and provides an easy way to differentiate different kit variants from one another (e.g., such as differentiating between kits for monitoring sleep, fitness, a particular sport, etc.). In some embodiments, kits are color-coded or have other artwork indicating their functionality. In other embodiments, kits have varying shapes and/or sizes as a result of their functionality (or alternatively to indicate their functionality). For example, the shape and sizing of patches included in kits may vary based on the intended use. As other examples, the number, functionality, shape, battery life or other features of hubs included in the kits may vary based on intended use. The carrying cases may also vary based on functionality of the hubs and/or patches (or more generally other devices used in physiologic monitoring of a subject) that are to be contained therein. For example, carrying cases may vary in battery life, size, shape, memory capacity (e.g., an amount of storage available for backing up or caching monitored physiologic data), storage capacity (e.g., an amount of hubs, patches or other devices which may be stored in the case), environmental weather-ability (e.g., with more or less insulation to account for varying temperature conditions, with more or less padding to protect from environments in which the carrying cases are subject to rough or sudden movement such as dropping, falling, crushing, etc.), or the like.

As discussed above, in some embodiments a carrying case includes a plurality of compartments, such as the chambers 201a, 201b in carrying case 200, chambers 301a, 301b in carrying case 300 and chambers 401a, 401b in carrying case 400. Such compartments or chambers may be openable via lids, sliding engagement with other portions of the carrying case, etc. In some embodiments, a first compartment of a carrying case includes mounting features such that one or more hubs may be mounted in the carrying case (e.g., mechanically mounted, magnetically mounted, etc.), while the second compartment includes space for storage of one or more patches for the attachment and interfacing of one or more of the hubs to a subject during use. Such hubs and patches are examples of reusable and disposable components of a device used in physiologic monitoring of a subject, respectively. It is to be appreciated, however, that such reusable and disposable components may take on other forms. For example, the disposable components may be a band, bracelet, necklace, sock, glove, hat or other clothing, glasses, headsets (e.g., such as virtual reality or augmented reality systems), components thereof, or the like. It is also to be appreciated that both portions of the device may be reusable in some embodiments, rather than just the hubs (e.g., patches or other form factors may be configured for multiple use or attachment to the subject).

The carrying case in some embodiments includes circuitry so as to charge a mounted hub, to calibrate one or more components within a mounted hub, to test the functionality of a mounted hub, or the like. In some embodiments, the carrying case may include one or more indicators (e.g., lights or other visual or audio indicators) to highlight to a user when one or more of the hubs are properly mounted/connected in the carrying case, to indicate whether one or more hubs are charging, to indicate a charge level of one or more hubs, to indicate faults or failures of one or more of the hubs, etc.

In some embodiments, the hubs and/or carrying case may include one or more microphones and/or speakers, so as to provide a personal assistant function, facilitate user annotation of a data stream or the like during use.

In some embodiments, one or more of the lids or other portions of the carrying case (e.g., such as portions of one or more of the chambers or compartments) may include a mirror. The mirror may facilitate attachment of a device to a target site on a body of the subject. For example, the top lid of a carrying case may be opened, with an interior portion thereof having a mirror that allows the subject to view a target site while mounting a hub, patch or other device thereupon. The mirror may be flat, and may have at least a portion thereof concave to magnify a view of a target site on the subject. FIG. 5 shows an example of a carrying case 500 that includes a mirror 505 on the interior of the top lid 503a. The carrying case 500, similar to carrying case 200, includes chambers 501a, 501b and lids 503a, 503b. Such an approach may be advantageous for facilitating self-application of patches and/or hubs onto one's body without requiring extra hands or a nearby mounted mirror. The mirror 505 of the carrying case 500 may allow a user to self-apply patches or hubs onto otherwise difficult-to-see regions of their body without the need for additional visual aids in the surrounding environment.

In some embodiments, the compartments of a carrying case are watertight when the lids thereof (or other access means) are closed. Such arrangements may be useful in remote environments, under hostile environmental conditions, etc.

In some embodiments, a carrying case may be configured with a plurality of externally-facing electrodes, along with internal connectors to connect such externally-facing or external electrodes to one or more hubs when the hubs are mounted in the carrying case. In these arrangements, when one or more hubs are mounted in the case and a user touches the external electrodes on the case, hardware within the hub may be coupled to the external electrodes via the connectors to monitor one or more physiologic parameters of the subject. As an example, the external electrodes may be configured to monitor electric field potentials on the subject between the external electrodes.

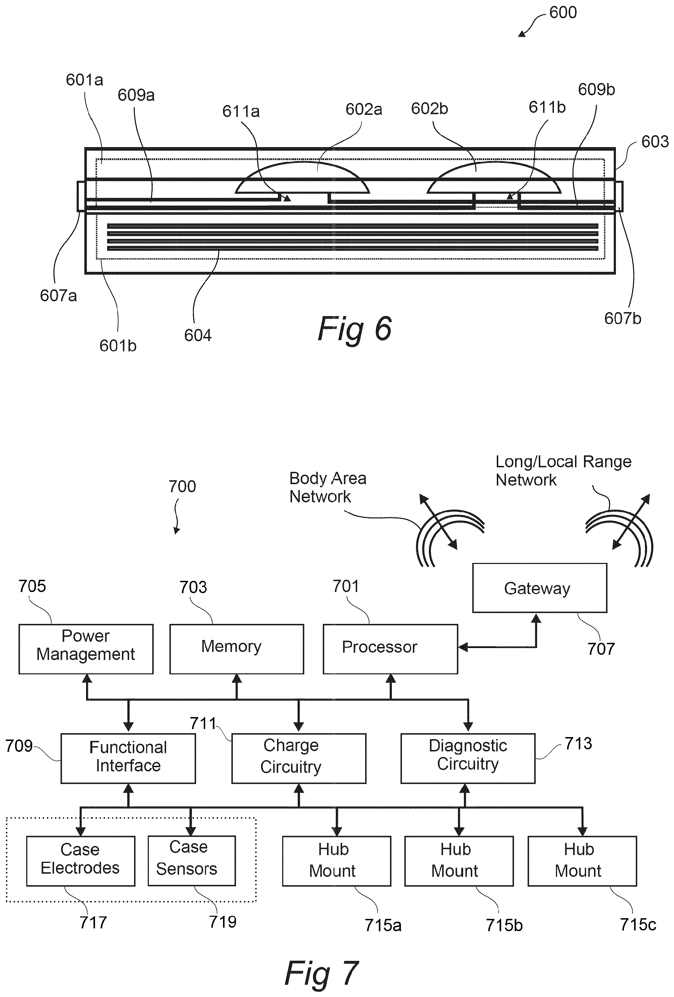

FIG. 6 illustrates an arrangement of a carrying case 600 including external electrodes 607a,b. As shown, the carrying case 600 has a form factor similar to that of carrying case 300, with chambers 601a, 601b and a top lid 603. Hubs 602a, 602b are mounted within the chamber 601a of the carrying case via respective hub mounts 611a, 611b. Wiring 609a connects the hub 602a to the electrodes 607a and 607b. Wiring 609b connects the hub 602b to the electrodes 607a and 607b. The wirings 609a, 609b are examples of internal connectors. A number of patches 604 are stored in chamber 601b.

External electrodes 607a, 607b may be connected to the hubs 602a, 602b via the wiring 609a, 609b and existing couplings that are on the bottom of the hubs 602a, 602b (e.g., magnetic or other mechanical and/or electrical couplings which may also be used to couple the hubs 602a, 602b to the patches 604). Sensors and circuitry within the hubs 602a, 602b, such as bioamplifiers, may thus be connected to the electrodes 607a, 607b via wiring 609a, 609b. Such an arrangement allows a user to quickly check, for example, hand-to-hand ECG when in the vicinity of the carrying case 600 by touching the electrodes 607a, 607b with their hands. Consider, for example, a usage scenario wherein a subject has the carrying case 600 in their purse or bag, and feels like they are having an attack or other medical event but isn't presently wearing one or more hub-patch pairs included in the carrying case 600. The subject may then quickly pull out the carrying case 600 and take a hand-to-hand reading. The carrying case 600 may also provide storage, memory or charging functionality as described elsewhere herein.

In some embodiments, carrying cases include local data storage or memory, which can provide a number of advantages such as alleviating required uptime on one or more networks, serving as a black box service, allowing for longer term data storage or caching while a remote server is unavailable, etc.

FIG. 7 shows a block diagram of a carrying case 700. As shown, the carrying case 700 includes a processor 701, memory 703, power management circuitry 705, gateway 707, functional interface 709, charge circuitry 711 and diagnostic circuitry 713, as well as hub mounts 715a, 715b, 715c and optional features such as case electrodes 717 and case sensors 719.

The processor 701 may comprise a microprocessor, a microcontroller, an application-specific integrated circuit (ASIC), a field-programmable gate array (FPGA) or other type of processing circuitry, as well as portions or combinations of such circuitry elements.

The memory 703 may comprise random access memory (RAM), read-only memory (ROM) or other types of memory, in any combination. The memory 703 and other memories disclosed herein may be viewed as examples of what are more generally referred to as "processor-readable storage media" storing executable computer program code or other types of software programs. Articles of manufacture comprising such processor-readable storage media are considered embodiments of the invention. A given such article of manufacture may comprise, for example, a storage device such as a storage disk, a storage array or an integrated circuit containing a memory. The processor 701 may load the computer program code from the memory 703 and execute the code to provide various functionalities described herein.

The power management circuitry 705 provides for managing power sources of the carrying case 700, and may include a battery, an energy harvesting system, or the like.

The gateway 707 provides network interface circuitry for communication with other devices on various types of networks, such as the BAN for management of and/or receiving physiologic monitoring data from devices attached to a subject as well as one or more local or long range networks for communication physiologic monitoring data to a remote server.

The functional interface 709 provides interconnection of various components within the carrying case 700.

Charge circuitry 711 provides for managing charging of hubs attached to the carrying case via hub mounts 715a, 715b, 715c. Charge circuitry 711 may also manage charging of the carrying case 700 itself, such as charging of a battery of the carrying case 700.

Diagnostic circuitry 713 is configured to perform diagnostics of hubs attached to the carrying case via hub mounts 715a, 715b, 715c. Such diagnostics may include checking for faults or failure of one or more of one or more hubs connected to the carrying case 700, as well as checking for faults or failure of other components within the carrying case 700.

Case electrodes 717 and case sensors 719 may be provided via connections to one or more hubs attached to the carrying case 700. In some embodiments, the case sensors 719 may include one or more microphones and/or one or more speakers.

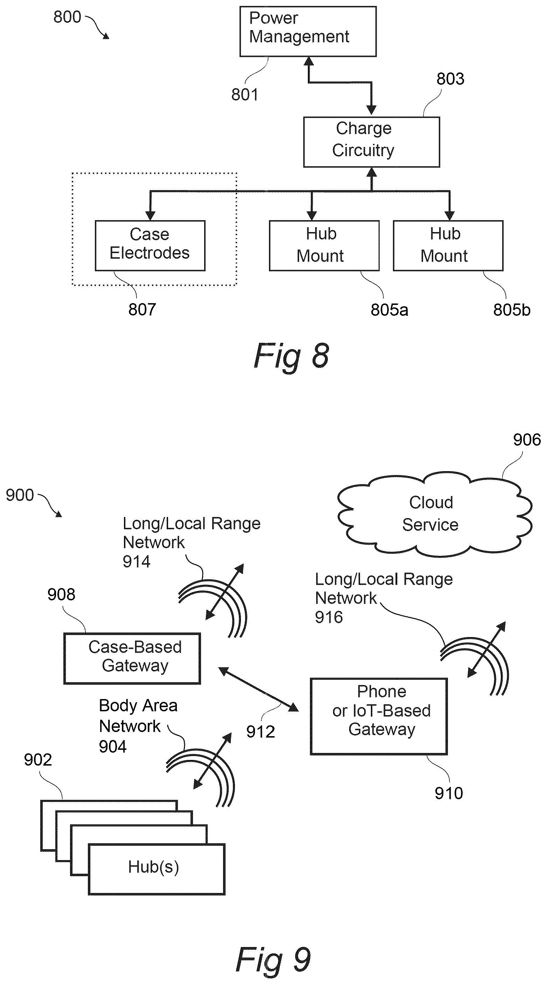

FIG. 8 shows another block diagram of a carrying case 800. The carrying case 800 includes power management circuitry 801, charge circuitry 803 and hub mounts 805a, 805b. The carrying case 800 also includes case electrodes 807, provided via connections to hubs attached to the case via hub mounts 805a, 805b. The power management circuitry 801 and charge circuitry provide functionality similar to that described above with respect to the power management circuitry 705 and charge circuitry 711.

FIG. 9 illustrates a system 900 including one or more hubs 902 that are configured for communication over a BAN 904. The hubs 902 may be attached to a subject and configured for communication on the BAN 904 with one another. The hubs 902 may be connected in a star or mesh configuration. The hubs 902 communicate with a remote server, illustratively implemented as cloud service 906, via gateways such as a case-based gateway 908 and/or a phone or IoT-based gateway 910. The case-based gateway 908 and phone or IoT-based gateway 910 facilitate communication of monitored physiologic data from the hubs 902 in the BAN 904 to the cloud service 906. Handoff 912 between the case-based gateway 908 and the phone or IoT-based gateway 910 can happen naturally based off wireless signal strength measurements between components of the system 900, as well as various other factors as detailed above. The case-based gateway 908 and phone or IoT-based gateway 910 communicate with the cloud service 906 via respective long or local range networks 914 and 916. The networks 914 and 916 may be, for example cellular networks, WiFi networks, etc.

In some usage scenarios, the case-based gateway 908 provides an access point that manages the BAN 904 and is a gateway to the cloud service 906. In such an arrangement, however, a subject to which the hubs 902 are attached may not want or be able to always carry or be in range of the carrying case implementing the case-based gateway at all times. Thus, when the subject is away from the carrying case implementing case-based gateway 908, a phone or other hand-held device can act as the BAN 904 manager by implementing the phone or IoT-based gateway 910.

In other usage scenarios, a subject may desire to designate their smartphone or another device implementing the phone or IoT-based gateway 910 as the primary gateway. A carrying case implementing the case-based gateway 908 may thus be configured as a bedside unit, which can hold hubs 902 and/or patches used to interface the hubs 902 to the subject, charge the hubs 902, and function as a night-time gateway so that the smartphone or other device implementing the phone or IoT-based gateway 910 is only used when the hubs 902 are out of wireless range of the carrying case implementing the case-based gateway 908. Various other usage scenarios are possible, and various logic or factors may be used in determining handoff between potential gateways. For example, state and connectivity in some embodiments may be managed through the cloud service 906.

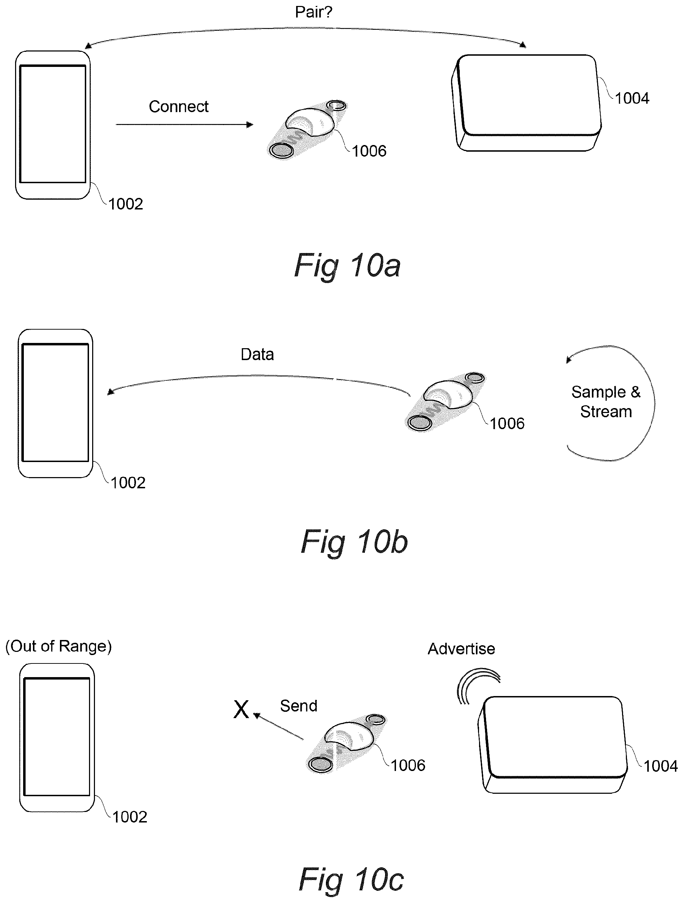

An example of a Bluetooth^{®} Low Energy (BLE) data flow for determining handoffs between a case-based gateway and a phone-based gateway will now be described with respect to FIGS. 10a-10m. It is to be appreciated, however, that BLE is just one example of a network protocol which may be used between gateways and devices in a BAN used for physiologic monitoring.

FIG. 10a illustrates a phone 1002 and a carrying case 1004 each configured to implement a respective gateway for a device 1006 configured for physiologic monitoring of a subject. The device 1006 is illustratively shown as a patch-hub pair, and is referred to below as a hub 1006. The phone 1002 and carrying case 1004 negotiate to determine which will connect to the hub 1006 and act as the gateway. Various factors may be used in making this decision, as well as other handoffs between gateways as described elsewhere herein. In the FIG. 10a example, the phone 1002 is the gateway and connects to the hub 1006. For clarity of illustration, only a single hub 1006 is shown, but embodiments are not limited to this arrangement - in other embodiments a plurality of hubs or other devices on a subject may be in communication with a gateway such as phone 1002 or carrying case 1004.

FIG. 10b illustrates operation of the phone 1002 as the gateway for the hub 1006. As shown, the hub 1006 conducts physiologic monitoring of a subject by sampling and streaming. The hub 1006 provides monitored physiologic data to the phone 1002, which may relay the monitored physiologic data to a remote server not explicitly shown in the figure.

Over time, the subject may move within an environment, possibly away from the phone 1002. In some cases, the hub 1006 on the subject is thereby taken out of range of the phone 1002. FIG. 10c illustrates such an arrangement, wherein the phone 1002 is out of range relative to the hub 1006. The hub 1006 attempts to send data to the phone 1002, but this fails as the phone 1002 is out of range. In this example, the carrying case 1004 is in range of the hub 1006 and is advertising its status as a potential gateway for the hub 1006.

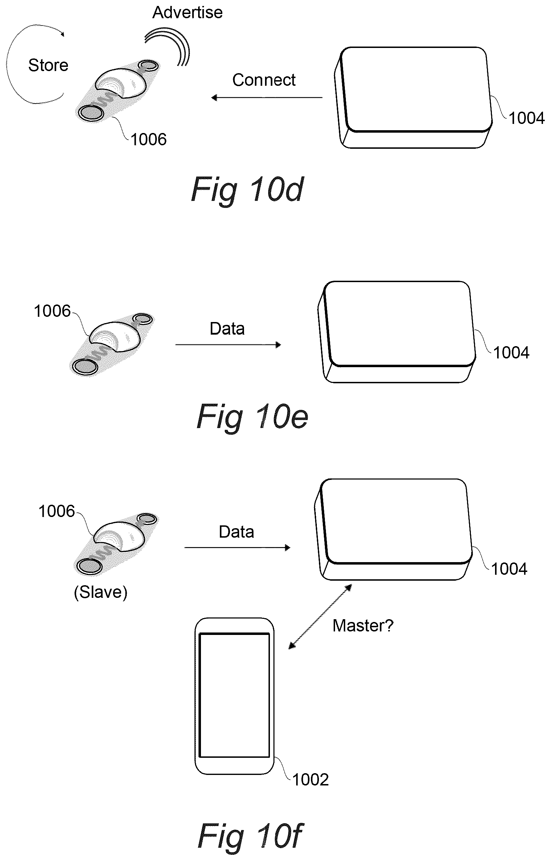

On detecting that the phone 1002 is out of range, the hub 1006 begins to store monitored physiologic data in an internal memory of the hub 1006 as illustrated in FIG. 10d. The hub 1006 also begins to advertise that it needs a gateway. As the carrying case 1004 is in range, the carrying case issues a connect command to the hub 1006. FIG. 10e illustrates that the hub 1006 then sends data, which may include stored data on the hub 1006 as well as newly monitored physiologic data, to the carrying case 1004 acting as the gateway.

As shown in FIG. 10f, the hub 1006 acts as a "slave" relative to the gateway acting as a "master" device in a master-slave BLE network architecture. When both the phone 1002 and the carrying case 1004 are in range of the hub 1006, the phone 1002 and carrying case 1004 negotiate to determine which will be the master device for the hub 1006. In this non-limiting example, the carrying case 1004 may switch back and forth between a central and peripheral operating mode, such that it can communicate to the phone 1002 as a BLE peripheral device, but to the hub 1006 as a BLE central device. FIG. 10f illustrates such an example, wherein the hub 1006 initially is sending data to the carrying case 1004 acting as the master device. When the phone 1002 also comes in range of the hub 1006, the phone 1002 and carrying case 1004 negotiate to determine which will be the master for the hub 1006.

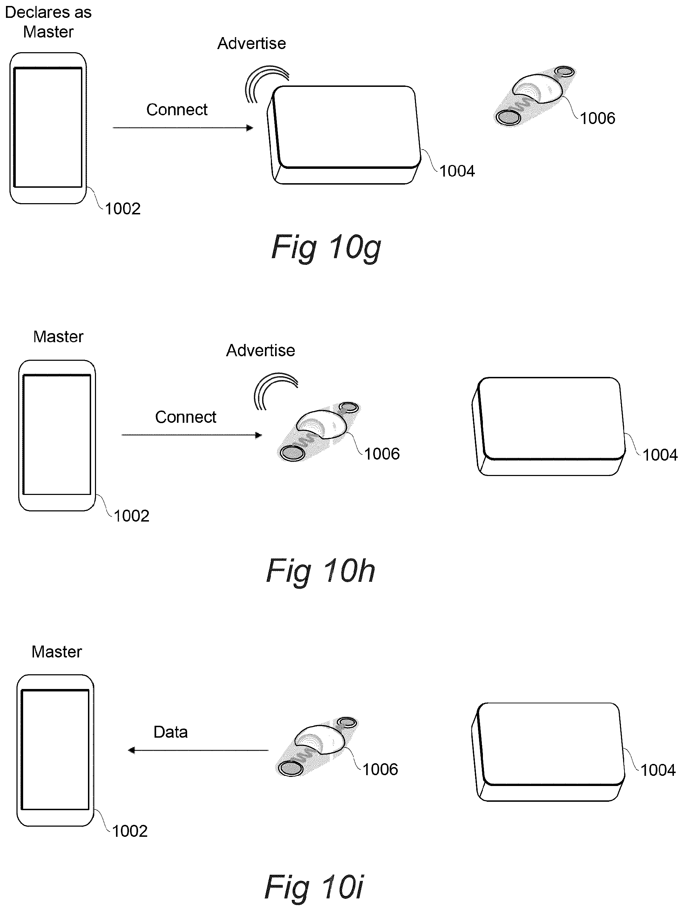

FIG. 10g shows an example wherein the phone 1002 declares as the master, and issues a connect command to the carrying case 1004. In response, the hub 1006 begins advertising its status looking for a new central device, and the carrying case 1004 establishes the session connection so as to provide the gateway for the hub 1006. This advantageously allows for the carrying case 1004 to discover and couple with the hub 1006 in the event that the phone 1002 becomes out or range, a handoff is desired, or some other type of event occurs.

FIG. 10h shows the phone 1002 issuing a connect command to the hub 1006, which is advertising its need for a gateway. After connecting, the hub 1006 begins sending data to the phone 1002 as shown in FIG. 10i.

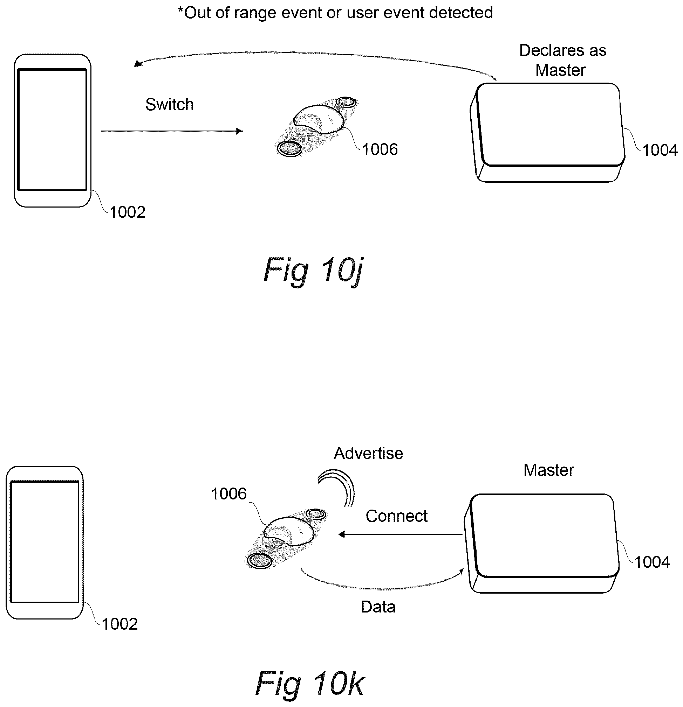

In response to detecting an out of range or other user event causing a handoff or other switch between gateways, a new device is set up as the BLE central device. FIG. 10j illustrates such a situation, where on detecting an out of range, low signal strength, or user event, the carrying case 1004 and phone 1002 establish that the carrying case 1004 will take over as the BLE central device going forward, and the hub 1006 is placed back into advertising mode and a wireless connection is established with the carrying case 1004. In FIG. 10j and other figures, the shorthand "master" is used to refer to a device being a BLE central device.

On receiving the switch command, the hub 1006 begins to advertise its need for a new gateway as shown in FIG. 10k. The carrying case 1004 responds by establishing a connection with the hub 1006. Thereafter, the hub 1006 sends data to the carrying case 1004 as the new BLE central device or gateway.

In one embodiment, the user may assign a permanent "master" (e.g., a wireless network master) for the connection that would act as gateway, using software on the smartphone. This approach may be advantageous to avoid the process of communication handoffs between the smartphone and the carrying case. In such embodiments, the permanent "master" may be set to either be the smartphone or the carrying case. Once assigned, the permanent master may take over management of the body area network whenever it is powered up and in range. Once a particular setup is established where the carrying case is the primary gateway and master for the associated body area network, the smartphone may still be paired with the carrying case for periodic communication. In such a situation, the carrying case may periodically update the smartphone regarding the status of one or more of the hubs (connection status, battery level etc.), one or more parameter collected on the body area network, or the like.

In one non-limiting example, the carrying case may route a first dataset from one or more of the hubs on the BAN through to a remote location (such as a cloud service, remote server, etc.) and communicate a subset of the first dataset, or one or more data sets derived therefrom to a secondary user device such as a smartphone, a watch, an earbud, or the like (e.g., such as streaming an electrocardiograph from the subject to a remote location while sending a sequence of heart-rate or r-r interval measurements derived from the ECG to the secondary device).

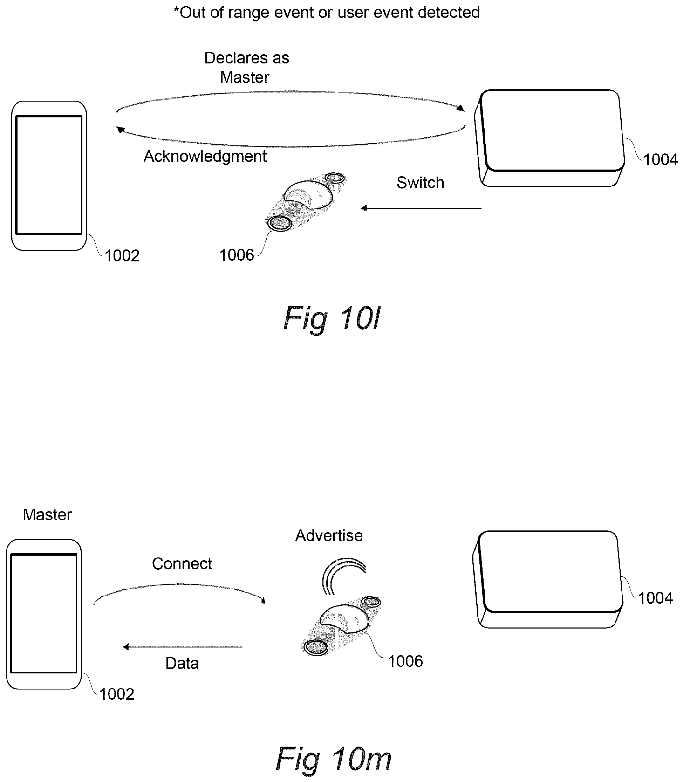

FIG. 10l illustrates another out of range or user event, which causes another handoff between gateways. The phone 1002 and carrying case 1004 negotiate a master status for the connection to the hub 1006 and, in this case, the phone 1002 takes over as master. The carrying case 1004 sends an acknowledgment to the phone 1002, and sends a switch command to the hub 1006.

As shown in FIG. 10m, the hub 1006 advertises its need for a new gateway. The phone 1002 then connects with the hub 1006 and starts a wireless session. Thereafter, the hub 1006 sends data to the phone 1002 as the new master device or gateway.

In another non-limiting example, the hub 1006 and the carrying case 1004 are connected to the phone 1002 as peripheral devices. The hub 1006 streams recorded data through the phone 1002, and the phone 1002 and carrying case 1004 communicate signal strength data between themselves and the hub 1006 to each other (e.g., between the hub 1006 and the phone 1002, between the hub 1006 and the case 1004, and between the case 1004 and the phone 1002). When one of the signal strengths is inadequate to maintain consistent wireless operation, the hub 1006 will begin storing data internally, and revert to an advertising mode, and phone 1002 and case 1004 will negotiate which device will take over as the central device. In one example, the case 1004 will take over and will revert to a central mode, and establish a connection with the hub 1006 so as to continue the data streaming process. In some non-limiting embodiments, the case 1004 may subsequently communicate such data to the phone 1002 or another gateway device (such as to a server over a WiFi network, etc.), as the wireless range of the case 1004 may be much higher than that of the hub 1002. In such an arrangement, the wireless signal strength of the hub 1006 may be lowered so as to save power, reduce nearfield signal exposure to a user, etc. and the secondary path (from the phone 1002 or case 1004) may be much longer range, given the ability in such devices to increase the antenna efficiency and wireless signal strength, to provide directed signals, etc. Such an arrangement may be advantageous to minimize power consumption on the hub 1006 during use.

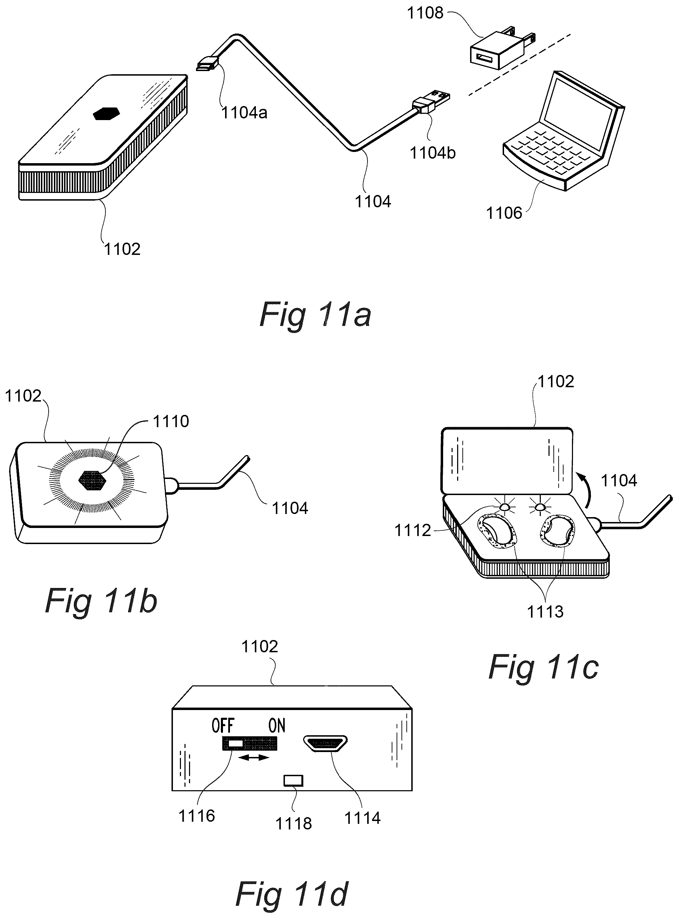

FIGs. 11a-11d illustrate how a carrying case 1102 may be charged. FIG. 11a shows the carrying case 1102 configured with a connector for connecting a charging cable 1104. The carrying case, for example, may include a female micro-Universal Serial Bus (USB) connector for attachment to male micro-USB connector 1104a of the charging cable 1104. The charging cable 1104 also includes a male USB 2.0+ connector 1104b, for connection to a device such as laptop computer 1106 or wall charger 1108.

FIG. 11b illustrates the carrying case 1102 with the charging cable 1104 plugged in. As shown, the carrying case 1102 includes an indicator 1110 configured to light up to indicate the charging status of the carrying case. For example, the indicator 1110 may include one or more light-emitting diodes (LEDs), configured to blink orange light when the carrying case 1102 is charging, and to provide a steady green light when the carrying case 1102 is fully charged. Various other combinations of colors, pulsing, etc. may be used to indicate charging status. In addition, a carrying case may be configured with multiple visual indicators, such that each visual indicator provides different information (e.g., to show charging status, to show a battery level of the carrying case, to show whether one or more hubs or other devices are mounted in the carrying case, to show whether the carrying case is acting as a gateway for one or more device, to show network connection status, etc.). In some embodiments, the carrying case 1102 may be configured with one or more audio and/or vibratory indicators configured to provide an auditory and/or vibratory indication in response to an event (e.g., connecting/disconnecting the charging cable 1104 from the carrying case 1102).

FIG. 11c illustrates the carrying case 1102 with a top lid thereof opened and two hubs 1113 mounted inside a compartment thereof. The charging cable 1104 is also connected to the carrying case 1102. As shown, the compartment includes indicators 1112, which may be LEDs, configured to provide charging indicators for the respective hubs mounted therein.

FIG. 11d illustrates an outside portion of the carrying case 1102, including a connector 1114 for connecting the charging cable 1104, a power switch 1116 and another indicator 1118. The power switch 1116 turns the carrying case 1102 on and off. The indicator 1118 may indicate whether the carrying case 1102 is powered on, whether a charging cable 1104 is connected to the connector 1114, etc.

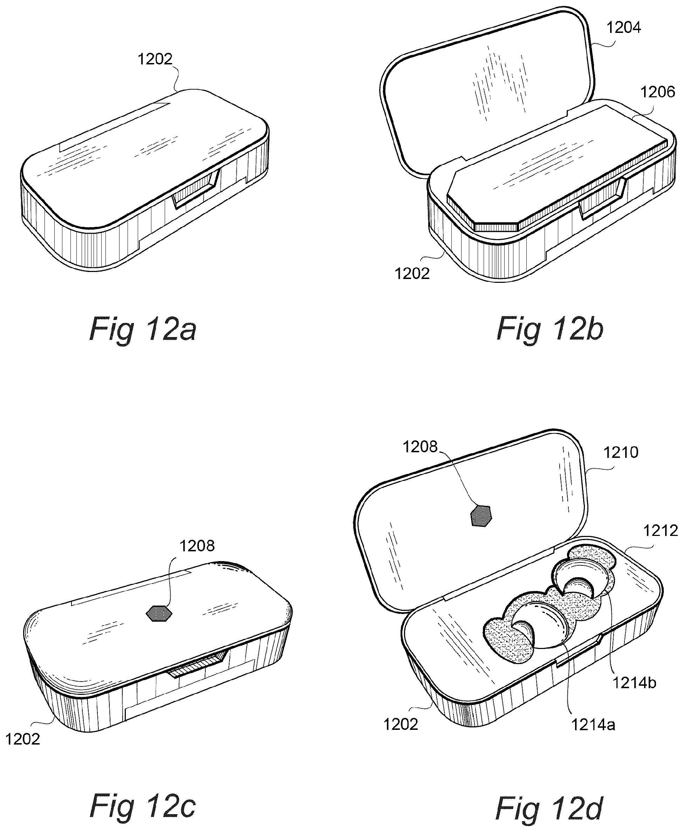

FIGS. 12a-12d illustrate a carrying case 1202. FIG. 12a shows a bottom side of the carrying case 1202. FIG. 12b shows the bottom lid 1204 of the carrying case 1202 opened, exposing a bottom chamber 1206 which may be used to store patches or other portions of a kit used in physiologic monitoring. FIG. 12c shows a top side of the carrying case 1202, including an indicator 1208. The indicator 1208, similar to indicator 1110, may be used to illustrate a charging status of the carrying case 1202 or various other information. FIG. 12d shows the top lid 1210 of the carrying case 1202 opened, exposing a top chamber 1212 with hubs 1214a, 1214b mounted in the top chamber 1212. The hubs 1214a, 1214b may be magnetically mounted in the top chamber 1212. The top side of the carrying case 1202 may, in addition to storing hubs, provide circuitry for various functions such as recharging, gateway, diagnostics, etc.

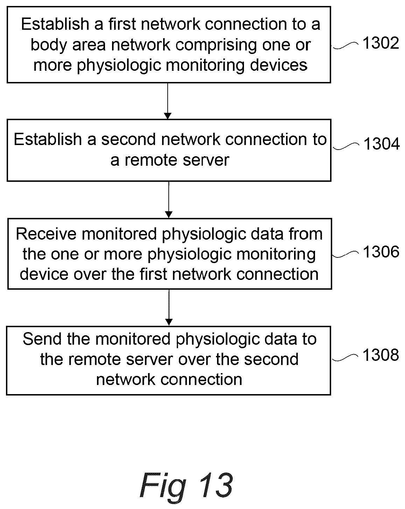

FIG. 13 shows a flow diagram of a process for using a carrying case as a gateway. The process comprises utilizing, in step 1302, a carrying case to establish a first network connection to a body area network comprising one or more devices attached to a subject and configured for monitoring one or more physiologic parameters of the subject. The process further comprises utilizing, in step 1304, the carrying case to establish a second network connection to a remote server. The process also includes receiving, in step 1306, monitored physiologic data at the carrying case from the one or more devices over the first network connection and sending, in step 1308, the monitored physiologic data from the carrying case to the remote server over the second network connection.

In some embodiments, the carrying case may include one or more diagnostic and/or calibration circuits configured to analyze the performance, operation or other characteristics of one or more attached hubs. The diagnostic and calibration circuits may be further configured to correct for calibration issues, to calibrate across multiple hubs, or the like. In one non-limiting example, the hubs include one or more orientation sensors and/or barometers. When the hubs are attached to the carrying case in their proper mounting locations, the distances and orientation between the hubs become fixed in space. Such information can be used to calibrate out offset in the barometers of two or more hubs (e.g., so as to automatically improve differential height measurements between the two or more hubs when later placed on a subject), to calibrate orientation of the accelerometers of two or more hubs (e.g., so as to ensure coordinated operation between multiple devices on a subject), and the like.

In some embodiments, two or more hubs may each include one or more temperature sensors. When attached to the carrying case, the two or more hubs may be at substantially the same temperature. Thus, temperature sensors on the two or more hubs may be calibrated by the carrying case when the two or more hubs are mounted therein, so that variations in temperature or temperature measurements by temperature sensors on the two or more hubs may be minimized during use. Such calibration can be used to improve multi-sensor skin temperature measurements and core temperature estimates on a subject during use.

In some embodiments, the calibration and diagnostic circuits of the carrying case may be configured with hardware for testing physiologic sensing hardware on hubs attached thereto. Such test hardware may include one or more lights for testing the function of a photodetector-based sensor (e.g., such as an SpO2 sensor on a hub), current or voltage function generator circuitry to test an electrophysiologic sensor on a hub, or the like. Such tests may be used to adjust for internal sensor drift and aging in the hubs, or to calibrate across multiple hubs in the system so as to improve system level precision and consistency during subsequent measurements on a subject.

The test hardware and/or calibration and diagnostic circuitry may be configured to determine if one or more hubs are meeting performance criteria for continued use on a subject. In the event that the test hardware and/or calibration and diagnostic circuitry determine that a hub or component thereof is defective, the carrying case may provide an indication to a user such that the hub is removed or replaced.

In some embodiments, carrying cases may be customized or designed for particular usage scenarios. In one non-limiting example, the usage scenario is a military usage scenario where a subject may be in a dangerous area. The carrying case may thus be configured with network interfaces or radios configured for burst mode operation, making it harder for an eavesdropper to identify a location of the subject or carrying case from signals emitted by the carrying case.

In another non-limiting example, the usage scenario is a movement analysis application and the kit includes a plurality of hubs configured for precision EMG and movement analysis on a subject. The carrying case may be customized to hold 2, 4, 6, 8, 12, 16 or more hubs, along with customized hardware to calibrate the movement-based functions of such hubs and to provide performance matching of the EMG hardware on the hubs. The kit may include multiple compartments for different patch types, where each patch type is sized for analyzing one or more muscle groups during use (e.g., potentially different patches for different muscle groups). Different patch types may include artwork or another indication of its function or suitability for particular muscle groups or for attachment to particular types of hubs.

In another non-limiting example, the usage scenario is a two-hub sleep apnea assessment application. A first hub is configured for placement onto the face, temple or forehead of a subject so as to monitor sleep parameters such as eye movement, EEG, breathing, facial, tongue or throat muscle activity, breathing sounds, swallowing sounds, choking sounds, or the like. A second hub is configured for placement onto the chest of the subject so as to monitor an electrocardiogram, heart sounds, lung sounds, respiratory depth, respiratory effort, SpO2, etc. The first and second hubs may, in a coordinated fashion, measure local movements, orientation differences between one other, or the like, so as to assess sleep state, sleep posture, or the like, in combination with physiologic parameters under test. The carrying case may include a plurality of patches of different types customized for use with either the first or second hub.

In another non-limiting example, the usage scenario is for a kit customized to generate a 12-lead equivalent electrocardiogram. The kit may include a plurality of hubs and patches, each configured for placement onto a particular site on the body of a subject. The carrying case for the kit is configured to manage the BAN between the hubs, such that precise timing and recording of data from each of the hubs can be collected during use and translated into an equivalent 12-lead ECG recording.

In some embodiments, the case may be configured so as to manage data transfer in conjunction with nearby IoT gateways such as WiFi gateways, personal assistant devices, or the like.

In some embodiments, carrying cases provide functionality for BAN management, such as managing one or more hubs simultaneously during use (e.g., when such hubs are attached to a subject. In one non-limiting example, the carrying case is configured to become a central device for a plurality of hubs on a subject, and a proprietary network may be established on the BAN so as to reduce power consumption, reduce data management overhead, increase data rates between devices, share timing information between devices (e.g., for simultaneous data collection applications), or the like. The carrying case may be configured to signal condition the data, compress the data, or the like before transferring the data or one or more signals related thereto to a remote host, cloud storage location, phone, watch, personal assistant-based device (e.g., for alerts, user interaction, etc.), a therapeutic device, or the like.

In some embodiments, a carrying case comprises at least one processing device comprising a processor coupled to a memory, at least one network interface, and a housing comprising at least one compartment, the at least one compartment comprising at least one mount configured for attachment of one or more devices utilized in physiologic monitoring of a subject. The at least one processing device is configured to utilize the at least one network interface to establish a first network connection to a body area network comprising the one or more devices, to utilize the at least one network interface to establish a second network connection to a remote server, to receive monitored physiologic data from the one or more devices over the first network connection, and to send the monitored physiologic data to the remote server over the second network connection.

In some embodiments, the first network connection comprises a short-range wireless network connection. The short-range wireless network connection may comprise a Bluetooth^{®} Low Energy network connection.

In some embodiments, the second network connection comprises a long-range wireless network connection. The long-range wireless network connection may comprise one of a cellular network connection and a WiFi network connection.

In some embodiments, the carrying case further comprises charging circuitry, the charging circuitry being configured to recharge at least a given one of the one or more devices when the given device is attached to the at least one mount. The carrying case may further comprise at least one indicator, the at least one indicator being configured to provide a visual indication of a charging status of the given device when the given device is attached to the at least one mount. The carrying case may further comprise a battery, the battery providing a power source for the charging circuitry to recharge the given device when the given device is attached to the at least one mount. The battery may have an energy storage capacity configured to recharge the given device at least five times.

In some embodiments, the carrying case further comprises at least one lid, the at least one lid being configured to open to expose at least a portion of the at least one compartment. The carrying case may further comprise a mirror on at least a portion of an interior surface of the at least one lid, the interior surface of the at least one lid being exposed when the at least one lid is opened.

In some embodiments, a given one of the one or more devices comprises a reusable component and a disposable component, the at least one compartment of the carrying case comprising a first compartment comprising the at least one mount for attachment of the reusable component and a second compartment for storage of the disposable component.

In some embodiments, the carrying case further comprises at least one electrode disposed on an outer surface of the carrying case and at least one interconnect coupling the at least one electrode to the at least one mount, wherein the at least one electrode is configured to provide monitored physiologic data to a given one of the one or more devices when the given device is attached to the at least one mount. The at least one electrode may comprise a first electrode disposed on a first portion of the outer surface of the carrying case and a second electrode disposed on a second portion of the outer surface of the carrying case. The first portion and the second portion may be arranged on the outer surface of the carrying case such that the first electrode is configured to interface with a first portion of the skin of a subject and the second electrode is configured to interface with a second portion of the skin of the subject. The at least one mount may comprise a first mount configured for attachment to a first one of the one or more devices and a second mount configured for attachment to a second one of the one or more devices. The at least one interconnect may comprise a first interconnect coupling the first and second electrodes to the first mount, and a second interconnect coupling the first and second electrodes to the second mount. The at least one interconnect may comprise a first interconnect coupling the first electrode to the first mount and a second interconnect coupling the second electrode to the second mount.

In some embodiments, the carrying case further comprises diagnostic circuitry configured to perform calibration of a given one of the one or more devices when the given device is attached to the at least one mount.

In some embodiments, the carrying case is configured as a gateway facilitating communication between the one or more other devices and the remote server, and the at least one processing device is further configured to determine whether to handoff between one or more other gateways configured to facilitate communication between the one or more devices and the remote server in response to an event. The event may comprise determining that at least one of the other gateways is in or out of range of the one or more devices in the body area network. The event may comprise determining a connection status of the one or more other gateways to the remote server.

In some embodiments, the at least one processing device is configured to store at least a portion of the monitored physiologic data in the memory. The at least one processing device may store the portion of the monitored physiologic data in the memory responsive to determining that the second network connection to the remote server is unavailable.

In some embodiments, a method comprises utilizing a carrying case to establish a first network connection to a body area network, the carrying case comprising a housing with at least one compartment, the at least one compartment comprising at least one mount configured for attachment of one or more devices utilized in physiologic monitoring of a subject, the body area network comprising the one or more devices. The method further comprises utilizing the carrying case to establish a second network connection to a remote server, receiving monitored physiologic data at the carrying case from the one or more devices over the first network connection, and sending the monitored physiologic data from the carrying case to the remote server over the second network connection.

In some embodiments, the first network connection comprises a short-range wireless network connection and the second network connection comprises a long-range wireless network connection.

In some embodiments, the method further comprises utilizing charging circuitry of the carrying case to recharge at least a given one of the one or more devices when the given device is attached to the at least one mount. The method may further comprise utilizing at least one indicator of the carrying case to provide a visual indication of a charging status of the given device.

In some embodiments, the method further comprises utilizing at least one electrode disposed on an outer surface of the case to provide monitored physiologic data to a given one of the one or more devices when the given device is attached to the at least one mount.

In some embodiments, the method further comprises utilizing diagnostic circuitry of the carrying case to perform calibration of a given one of the one or more devices when the given device is attached to the at least one mount.

In some embodiments, the carrying case is configured as a gateway facilitating communication between the one or more devices and the remote server, and the method further comprises determining whether to handoff between one or more other gateways configured to facilitate communication between the one or more devices and the remote server in response to an event. The event may comprise determining that at least one of the other gateways is in or out of range of the one or more devices in the body area network. The event may comprise determining a connection status of the one or more other gateways to the remote server.

In some embodiments, the method further comprises storing at least a portion of the monitored physiologic data in a memory of the carrying case. Storing the portion of the monitored physiologic data in the memory may be responsive to determining that the second network connection to the remote server is unavailable.

In some embodiments, a physiologic monitoring kit comprises a carrying case comprising one or more mounts and a housing comprising one or more compartments, one or more physiologic monitoring devices configured for mechanical interconnection with the one or more mounts, and one or more subject interfaces configured to fit into one of the compartments and configured to mechanically interface with one or more of the physiologic monitoring devices.

In some embodiments, the carrying case of the kit comprises a power source coupled to the mounts, the carrying case being configured to recharge a given one of the physiologic monitoring devices when the given physiologic monitoring device is coupled to at least one of the mounts.

In some embodiments, the carrying case of the kit comprises at least a first radio, the carrying case being configured to utilize the first radio for wireless communication with the one or more physiologic monitoring devices to receive monitored physiologic data therefrom. The carrying case of the kit may further comprise a second radio, the carrying case being configured to utilize the second radio for wireless communication with at least one of a satellite and a radio tower to send the monitored physiologic data from the one or more physiologic monitoring devices to a remote server. The carrying case may further comprise a subscriber identity module for a cellular network, the carrying case being configured to utilize the subscriber identity module for wireless communication over the cellular network to send the monitored physiologic data from the one or more physiologic monitoring devices to a remote server.

In some embodiments, the mounts of the carrying case are disposed within one or more of the compartments of the carrying case, the one or more compartments configured to be sealed such that the mounts are isolated from a surrounding environment.

In some embodiments, one or more of the compartments of the carrying case comprise a lid, at least a portion of an interior face of the lid being lined with a mirror such that a subject holding the carrying case can view an attachment site on a body of the subject when attaching one or more of the subject interfaces to the attachment site or when attaching one or more of the physiologic monitoring devices to one or more of the subject interfaces.

In some embodiments, the carrying case of the kit comprises two or more electrodes disposed on an outer surface thereof, the two or more electrodes being electrically coupled to the one or more mounts such that one or more of the physiologic monitoring devices are coupled to at least one of the two or more electrodes when the one or more physiologic monitoring devices are attached to the one or more mounts.

In some embodiments, the carrying case of the kit comprises a microphone and a speaker, and the carrying case is configured to utilize the microphone and the speaker to receive communications and provide feedback to a subject during use.

In some embodiments, the kit further comprises a therapeutic device, at least one of the carrying case and one or more of the physiologic monitoring devices comprising a radio configured for communication with the therapeutic device.

It will be appreciated that additional advantages and modifications will readily occur to those skilled in the art. Therefore, the disclosures presented herein and broader aspects thereof are not limited to the specific details and representative embodiments shown and described herein.

## Claims

1. A physiologic monitoring kit comprising:
a carrying case (200; 300; 400; 500; 600; 700; 800; 908; 1004; 1102; 1202) comprising one or more mounts (611a; 611b; 715a; 715b; 715c; 805a; 805b) and a housing comprising one or more compartments (201a; 201b; 301a; 301b; 401a; 401b; 501a; 501b; 601a; 601b; 1206; 1212);
one or more physiologic monitoring devices (302; 304; 402; 404; 602a; 602b; 902; 1006; 1113; 1214a; 1214b) configured for mechanical interconnection with the one or more mounts; and
one or more subject interfaces (304; 404; 604) configured to fit into one of the compartments and configured to mechanically interface with one or more of the physiologic monitoring devices;
wherein the carrying case is configured to act as a gateway facilitating communication between (i) at least a subset of the one or more physiologic monitoring devices that are part of a body area network (904) associated with a subject and (ii) a remote server (906); and
**characterised in that**
the carrying case is configured to control handoff to one or more other gateways configured to facilitate communication between the one or more physiologic monitoring devices and the remove server, wherein the handoff causes the subset of the one or more physiologic monitoring devices that are part of the body area network to send monitored physiologic data to at least one of the one or more other gateways instead of the carrying case.

2. The kit of claim 1, wherein the carrying case comprises a power source (705, 711; 801, 803) coupled to the mounts.

3. The kit of claim 2, wherein the carrying case is configured to recharge a given one of the physiologic monitoring devices when the given physiologic monitoring device is coupled to at least one of the mounts.

4. The kit of claim 1, wherein the carrying case comprises at least a first radio, the carrying case being configured to utilize the first radio for wireless communication with the subset of the one or more physiologic monitoring devices that are in the body area network to receive monitored physiologic data therefrom.

5. The kit of claim 4, wherein the carrying case comprises a second radio, the carrying case being configured to utilize the second radio for wireless communication with at least one of a satellite and a radio tower to send the monitored physiologic data from the subset of the one or more physiologic monitoring devices to the remote server.

6. The kit of claim 4, wherein the carrying case comprises a subscriber identity module for a cellular network, the carrying case being configured to utilize the subscriber identity module for wireless communication over the cellular network to send the monitored physiologic data from the subset of the one or more physiologic monitoring devices to the remote server.

7. The kit of claim 1, wherein the mounts are disposed within one or more of the compartments.

8. The kit of claim 7, wherein the one or more compartments are configured to be sealed such that the mounts are isolated from a surrounding environment.

9. The kit of claim 1, wherein one or more of the compartments comprises a lid (203a; 203b; 303; 403a; 403b; 503a; 503b; 603; 1204; 1210).

10. The kit of claim 9, wherein at least a portion of an interior face of the lid is lined with a mirror (505) such that the subject holding the carrying case can view an attachment site on a body of the subject when attaching one or more of the subject interfaces to the attachment site or when attaching one or more of the physiologic monitoring devices to one or more of the subject interfaces.

11. The kit of claim 1, wherein the carrying case comprises two or more electrodes (607a; 607b; 717; 807) disposed on an outer surface thereof.

12. The kit of claim 11, wherein the two or more electrodes are electrically coupled to the one or more mounts such that one or more of the physiologic monitoring devices are coupled to at least one of the two or more electrodes when the one or more physiologic monitoring devices are attached to the one or more mounts.

13. The kit of claim 1, wherein the carrying case comprises a microphone and a speaker, and the carrying case is configured to utilize the microphone and the speaker to receive communications and provide feedback to a subject during use.

14. The kit of claim 1, further comprising a therapeutic device (302; 304; 402; 404; 602a; 602b; 902; 1006; 1113; 1214a; 1214b).

15. The kit of claim 14, wherein at least one of the carrying case and one or more of the physiologic monitoring devices comprises a radio configured for communication with the therapeutic device.

## Patentansprüche

1. Physiologisches Überwachungskit, umfassend:
einen Tragekoffer (200; 300; 400; 500; 600; 700; 800; 908; 1004; 1102; 1202), der eine oder mehrere Halterungen (611a; 611b; 715a; 715b; 715c; 805a; 805b) und ein Gehäuse umfasst, das ein oder mehrere Fächer (201a; 201b; 301a; 301b; 401a; 401b; 501a; 501b; 601a; 601b; 1206; 1212) umfasst;
eine oder mehrere physiologische Überwachungsvorrichtungen (302; 304; 402; 404; 602a; 602b; 902; 1006; 1113; 1214a; 1214b), die für eine mechanische Zwischenverbindung mit der einen oder den mehreren Halterungen konfiguriert sind; und
eine oder mehrere Subjektschnittstellen (304; 404; 604), die konfiguriert sind, um in eines der Fächer zu passen, und die konfiguriert sind, um mechanisch mit einer oder mehreren der physiologischen Überwachungsvorrichtungen zu verbinden;
wobei der Tragekoffer konfiguriert ist, um als Gateway zu fungieren, das die Kommunikation zwischen (i) mindestens einem Teilsatz der einen oder mehreren physiologischen Überwachungsvorrichtungen, die Teil eines einem Subjekt zugeordneten Körperbereichsnetzwerks (904) sind, und (ii) einem Fernserver (906) erleichtert; und
**dadurch gekennzeichnet, dass**
der Tragekoffer konfiguriert ist, um die Weitergabe an ein oder mehrere andere Gateways zu steuern, die konfiguriert sind, um die Kommunikation zwischen der einen oder den mehreren physiologischen Überwachungsvorrichtungen und dem Entfernungsserver zu erleichtern, wobei die Weitergabe bewirkt, dass der Teilsatz der einen oder mehreren physiologischen Überwachungsvorrichtungen, die Teil des Körperbereichsnetzwerks sind, überwachte physiologische Daten an mindestens eines der einen oder mehreren anderen Gateways anstelle des Tragekoffers sendet.

2. Kit nach Anspruch 1, wobei der Tragekoffer eine mit den Halterungen gekoppelte Energiequelle (705, 711; 801, 803) umfasst.

3. Kit nach Anspruch 2, wobei der Tragekoffer konfiguriert ist, um eine bestimmte der physiologischen Überwachungsvorrichtungen aufzuladen, wenn die bestimmte physiologische Überwachungsvorrichtung an mindestens eine der Halterungen gekoppelt ist.

4. Kit nach Anspruch 1, wobei der Tragekoffer mindestens ein erstes Funkgerät umfasst, wobei der Tragekoffer konfiguriert ist, um das erste Funkgerät für die drahtlose Kommunikation mit dem Teilsatz der einen oder mehreren physiologischen Überwachungsvorrichtungen zu verwenden, die sich in dem Körperbereichsnetzwerk befinden, um überwachte physiologische Daten von dort zu empfangen.

5. Kit nach Anspruch 4, wobei der Tragekoffer ein zweites Funkgerät umfasst, wobei der Tragekoffer konfiguriert ist, um das zweite Funkgerät zur drahtlosen Kommunikation mit mindestens einem von einem Satelliten und einem Funkturm zu verwenden, um die überwachten physiologischen Daten von dem Teilsatz der einen oder mehreren physiologischen Überwachungsvorrichtungen an den Fernserver zu senden.

6. Kit nach Anspruch 4, wobei der Tragekoffer ein Teilnehmeridentitätsmodul für ein Mobilfunknetzwerk umfasst, wobei der Tragekoffer konfiguriert ist, um das Teilnehmeridentitätsmodul für die drahtlose Kommunikation über das Mobilfunknetzwerk zu verwenden, um die überwachten physiologischen Daten von dem Teilsatz der einen oder mehreren physiologischen Überwachungsvorrichtungen an den Fernserver zu senden.

7. Kit nach Anspruch 1, wobei die Halterungen in einem oder mehreren der Fächer angeordnet sind.

8. Kit nach Anspruch 7, wobei das eine oder die mehreren Fächer konfiguriert sind, um so abgedichtet zu werden, dass die Halterungen von einer umliegenden Umgebung isoliert sind.

9. Kit nach Anspruch 1, wobei eines oder mehrere der Fächer einen Deckel (203a; 203b; 303; 403a; 403b; 503a; 503b; 603; 1204; 1210) umfassen.

10. Kit nach Anspruch 9, wobei mindestens ein Abschnitt einer inneren Fläche des Deckels mit einem Spiegel (505) ausgekleidet ist, sodass das Subjekt, das den Tragekoffer hält, eine Anbringungsstelle an einem Körper des Subjekts sehen kann, wenn es eine oder mehrere der Subjektschnittstellen an der Anbringungsstelle anbringt oder wenn es eine oder mehrere der physiologischen Überwachungsvorrichtungen an einer oder mehreren der Subjektschnittstellen anbringt.

11. Kit nach Anspruch 1, wobei der Tragekoffer zwei oder mehr Elektroden (607a; 607b; 717; 807) umfasst, die auf einer äußeren Oberfläche davon angeordnet sind.

12. Kit nach Anspruch 11, wobei die zwei oder mehr Elektroden elektrisch mit der einen oder den mehreren Halterungen gekoppelt sind, sodass eine oder mehrere der physiologischen Überwachungsvorrichtungen mit mindestens einer der zwei oder mehreren Elektroden gekoppelt sind, wenn die eine oder mehreren physiologischen Überwachungsvorrichtungen an der einen oder den mehreren Halterungen befestigt sind.

13. Kit nach Anspruch 1, wobei der Tragekoffer ein Mikrofon und einen Lautsprecher umfasst und der Tragekoffer konfiguriert ist, um das Mikrofon und den Lautsprecher zu verwenden, um Kommunikationen zu empfangen und einem Subjekt während der Verwendung eine Rückmeldung bereitzustellen.

14. Kit nach Anspruch 1, ferner umfassend eine therapeutische Vorrichtung (302; 304; 402; 404; 602a; 602b; 902; 1006; 1113; 1214a; 1214b).

15. Kit nach Anspruch 14, wobei mindestens eine der Tragetasche und eine oder mehrere der physiologischen Überwachungsvorrichtungen ein Funkgerät umfasst, das für die Kommunikation mit der therapeutischen Vorrichtung konfiguriert ist.

## Revendications

1. Kit de surveillance physiologique comprenant :
un étui de transport (200 ; 300 ; 400 ; 500 ; 600 ; 700 ; 800 ; 908 ; 1004 ; 1102 ; 1202) comprenant un ou plusieurs supports (611a ; 611b ; 715a ; 715b ; 715c ; 805a ; 805b) et un boîtier comprenant un ou plusieurs compartiments (201a ; 201b ; 301a ; 301b ; 401a ; 401b ; 501a ; 501b ; 601a ; 601b ; 1206 ; 1212) ;
un ou plusieurs dispositifs de surveillance physiologique (302 ; 304 ; 402 ; 404 ; 602a ; 602b ; 902 ; 1006 ; 1113 ; 1214a ; 1214b) configurés pour une interconnexion mécanique avec les un ou plusieurs supports ; et
une ou plusieurs interfaces de sujet (304 ; 404 ; 604) configurées pour s'insérer dans l'un des compartiments et configurées pour s'interfacer mécaniquement avec un ou plusieurs des dispositifs de surveillance physiologique ;
dans lequel l'étui de transport est configuré pour servir de passerelle facilitant la communication entre (i) au moins un sous-ensemble des un ou plusieurs dispositifs de surveillance physiologique qui font partie d'un réseau corporel (904) associé à un sujet et (ii) un serveur distant (906) ; et
**caractérisé en ce que**
l'étui de transport est configuré pour commander le transfert vers une ou plusieurs autres passerelles configurées pour faciliter la communication entre les un ou plusieurs dispositifs de surveillance physiologique et le serveur distant, dans lequel le transfert amène le sous-ensemble des un ou plusieurs dispositifs de surveillance physiologique qui font partie du réseau corporel à envoyer des données physiologiques surveillées à au moins l'une des une ou plusieurs autres passerelles au lieu de l'étui de transport.

2. Kit selon la revendication 1, dans lequel l'étui de transport comprend une source d'alimentation (705, 711 ; 801, 803) couplée aux supports.

3. Kit selon la revendication 2, dans lequel l'étui de transport est configuré pour recharger un dispositif donné des dispositifs de surveillance physiologique lorsque le dispositif de surveillance physiologique donné est couplé à au moins l'un des supports.

4. Kit selon la revendication 1, dans lequel l'étui de transport comprend au moins une première radio, l'étui de transport étant configuré pour utiliser la première radio pour communiquer sans fil avec le sous-ensemble des un ou plusieurs dispositifs de surveillance physiologique qui se trouvent dans le réseau corporel pour recevoir des données physiologiques surveillées à partir de celui-ci.

5. Kit selon la revendication 4, dans lequel l'étui de transport comprend une seconde radio, l'étui de transport étant configuré pour utiliser la seconde radio pour communiquer sans fil avec au moins l'un d'un satellite et d'une tour radio pour envoyer les données physiologiques surveillées du sous-ensemble des un ou plusieurs dispositifs de surveillance physiologique au serveur distant.

6. Kit selon la revendication 4, dans lequel l'étui de transport comprend un module d'identification d'abonné pour un réseau cellulaire, l'étui de transport étant configuré pour utiliser le module d'identification d'abonné pour communiquer sans fil sur le réseau cellulaire pour envoyer les données physiologiques surveillées du sous-ensemble des un ou plusieurs dispositifs de surveillance physiologique au serveur distant.

7. Kit selon la revendication 1, dans lequel les supports sont disposés dans un ou plusieurs des compartiments.

8. Kit selon la revendication 7, dans lequel les un ou plusieurs compartiments sont configurés pour être scellés de telle sorte que les supports sont isolés d'un environnement ambiant.

9. Kit selon la revendication 1, dans lequel un ou plusieurs des compartiments comprennent un couvercle (203a ; 203b ; 303 ; 403a ; 403b ; 503a ; 503b ; 603 ; 1204 ; 1210).

10. Kit selon la revendication 9, dans lequel au moins une partie d'une face intérieure du couvercle est revêtue d'un miroir (505) de telle sorte que le sujet tenant l'étui de transport peut voir un site de fixation sur un corps du sujet lors de la fixation d'une ou plusieurs des interfaces de sujet au site de fixation ou lors de la fixation d'un ou plusieurs des dispositifs de surveillance physiologique à une ou plusieurs des interfaces de sujet.

11. Kit selon la revendication 1, dans lequel l'étui de transport comprend deux ou plusieurs électrodes (607a ; 607b ; 717 ; 807) disposées sur une surface extérieure de celui-ci.

12. Kit selon la revendication 11, dans lequel les deux ou plusieurs électrodes sont couplées électriquement aux un ou plusieurs supports de telle sorte qu'un ou plusieurs des dispositifs de surveillance physiologique sont couplés à au moins l'une des deux ou plusieurs électrodes lorsque les un ou plusieurs dispositifs de surveillance physiologique sont fixés aux un ou plusieurs supports.

13. Kit selon la revendication 1, dans lequel l'étui de transport comprend un microphone et un haut-parleur, et l'étui de transport est configuré pour utiliser le microphone et le haut-parleur pour recevoir des communications et fournir une rétroaction à un sujet lors de l'utilisation.

14. Kit selon la revendication 1, comprenant en outre un dispositif thérapeutique (302 ; 304 ; 402 ; 404 ; 602a ; 602b ; 902 ; 1006 ; 1113 ; 1214a ; 1214b).

15. Kit selon la revendication 14, dans lequel au moins l'un de l'étui de transport et d'un ou plusieurs des dispositifs de surveillance physiologique comprennent une radio configurée pour communiquer avec le dispositif thérapeutique.
